(19) Europäisches Patentamt
European Patent Office
Office européen des brevets

(11) EP 3 166 917 B1

(12) **FASCICULE DE BREVET EUROPEEN**

(45) Date de publication et mention de la délivrance du brevet:
**21.04.2021 Bulletin 2021/16**

(21) Numéro de dépôt: **15739008.9**

(22) Date de dépôt: **17.06.2015**

(51) Int Cl.:
*C07C 37/055* (2006.01) *C07C 39/11* (2006.01)
*C07C 39/08* (2006.01) *C05F 11/00* (2006.01)
*C08H 7/00* (2011.01) *C07F 7/18* (2006.01)

(86) Numéro de dépôt international:
**PCT/IB2015/054570**

(87) Numéro de publication internationale:
**WO 2016/005837 (14.01.2016 Gazette 2016/02)**

(54) **PROCEDE DE PREPARATION DE COMPOSES AROMATIQUES A PARTIR DE LA LIGNINE**

VERFAHREN ZUR HERSTELLUNG VON AROMATISCHEN VERBINDUNGEN AUS LIGNIN

METHOD OF PREPARING AROMATIC COMPOUNDS FROM LIGNIN

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **09.07.2014 FR 1456638**

(43) Date de publication de la demande:
**17.05.2017 Bulletin 2017/20**

(73) Titulaire: **Commissariat à l'Énergie Atomique et aux Énergies Alternatives
75015 Paris (FR)**

(72) Inventeurs:
- **FEGHALI, Elias
Springfield, 3015 Rotorua (NZ)**
- **CANTAT, Thibault
F-92130 Issy Les Moulineaux (FR)**

(74) Mandataire: **Gevers & Orès
Immeuble le Palatin 2
3 Cours du Triangle
CS 80165
92939 Paris La Défense Cedex (FR)**

(56) Documents cités:
**WO-A2-2011/003029**

- **JIANFENG ZHANG ET AL: "Reductive Degradation of Lignin and Model Compounds by Hydrosilanes", ACS SUSTAINABLE CHEMISTRY & ENGINEERING, vol. 2, no. 8, 3 juillet 2014 (2014-07-03) , pages 1983-1991, XP055171789, ISSN: 2168-0485, DOI: 10.1021/sc500302j**
- **ELIAS FEGHALI ET AL: "Unprecedented organocatalytic reduction of lignin model compounds to phenols and primary alcohols using hydrosilanes", CHEMICAL COMMUNICATIONS, vol. 50, no. 7, 1 janvier 2014 (2014-01-01), pages 862-865, XP055171824, ISSN: 1359-7345, DOI: 10.1039/C3CC47655C cité dans la demande**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 3 166 917 B1

## Description

La présente invention concerne un procédé de préparation de composés aromatiques monocycliques à partir de la lignine et l'utilisation dudit procédé et/ou desdits composés aromatiques obtenus par le procédé de l'invention dans la fabrication de combustibles, de composants électroniques, de polymères plastiques, de caoutchouc, de médicaments, de vitamines, de produits cosmétiques, de parfums, de produits alimentaires, de fils et fibres synthétiques, de cuirs synthétiques, de colles, de pesticides, d'engrais.

Elle concerne également un procédé de fabrication de combustibles, de composants électroniques, de polymères plastiques, de caoutchouc, de médicaments, de vitamines, de produits cosmétiques, de parfums, de produits alimentaires de fils et fibres synthétiques, de cuirs synthétiques, de colles, de pesticides, d'engrais, comprenant une étape de préparation de composés aromatiques à partir de la lignine par le procédé selon l'invention.

Le bois est composé de trois constituants majeurs : la cellulose, l'hémicellulose et la lignine. La cellulose et l'hémicellulose sont déjà valorisées dans l'industrie, en particulier dans l'industrie papetière. Cette utilisation génère chaque année plusieurs millions de tonnes de sous-produits riches en lignine qui sont utilisés comme combustibles de faible capacité calorifique pour fournir chaleur et énergie aux procédés de production du papier. En parallèle, une quantité minime de la lignine est isolée par extraction directe à partir de plantes (F. G. Calvo-Flores et J. A. Dobado, ChemSusChem. 2010, 3, pages 1227-1235).

La lignine est la substance la plus abondante en terme de source de groupements aromatiques dans la nature et le plus grand contributeur au sol de la matière organique (S. Y. Lin, in Methods in Lignin Chemistry, Springer Series in Wood Science (Ed.: C. W. Dence), Springer, Berlin 1992). Elle résulte essentiellement de la polymérisation radicalaire de trois monomères nommés monolignols : l'alcool p-coumarilyque, l'alcool coniférylique, et l'alcool sinapylique, qui après une polymérisation par déshydrogénation avec la péroxydase donnent respectivement les résidus p-hydroxyphényle (H), guaiacyle (G), et syringyl (S) comme illustré dans la Figure 1 ci- dessous (R. Vanholme, K. Morreel, J. R. W. Boerjan, Curr. Opin. Plant Biol. 2008, 11, pages 278-285) :

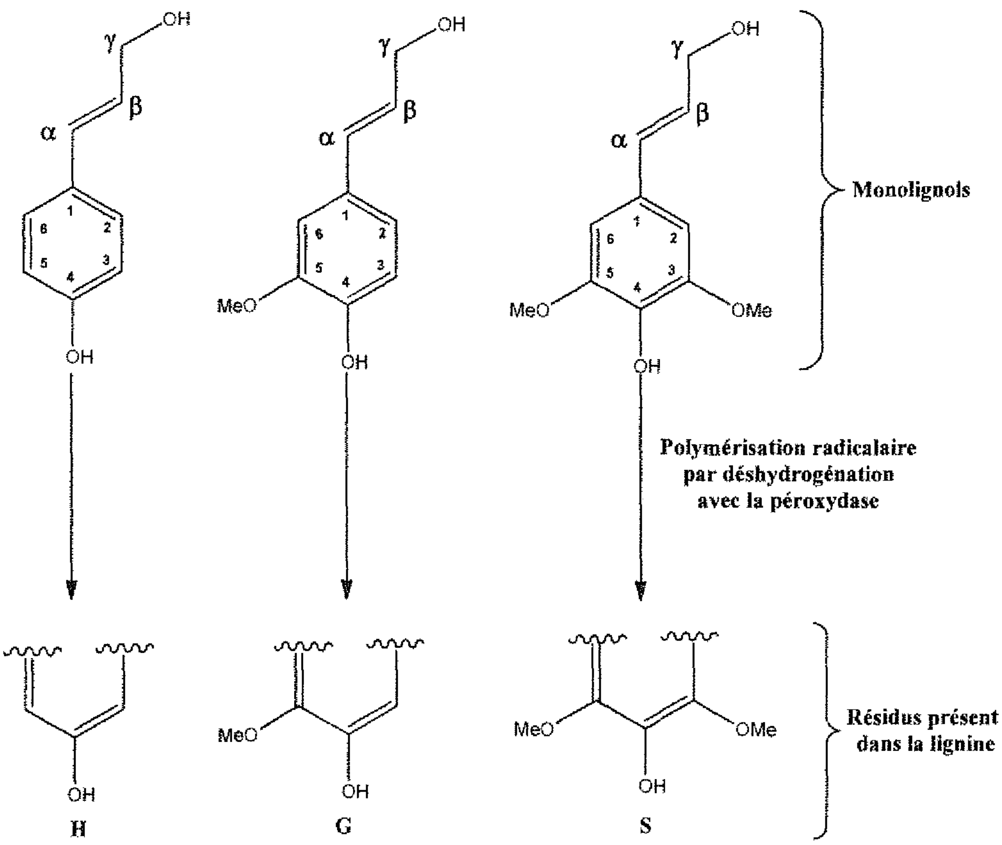


Figure 1

La complexité ainsi que la diversité de la structure de la lignine est largement dépendante de son origine. En se basant sur la taxonomie végétale, il a été proposé que la lignine issue des gymnospermes (appelés « bois tendre » ou « softwood » en anglais) présente plus de résidus G, que celle issue des angiospermes (appelés « bois dur » ou

hardwood en anglais), qui contient un mélange de résidus G et S, et la lignine issue des plantes herbacées contient un mélange des trois résidus aromatiques H, G et S. Une technique de classification plus rigoureuse a été de se baser sur une approche chimique dans laquelle les lignines sont classées selon l'abondance des motifs G, H et S dans le polymère. Quatre grands groupes de lignine ont ainsi été identifiés : le type G, le type-GS, le type HGS et le type HG (F. G. Calvo-Flores et J. A. Dobado, ChemSusChem. 2010, 3, pages 1227-1235).

Quel que soit le type de lignine, ce bio-polymère se caractérise par une grande hétérogénéité chimique et est constitué d'unités propyl-phénol liées entre elles par l'intermédiaire de divers types de liaisons C-O et C-C de type aryle éther, aryle glycérol et β-aryle éther. La Figure 2 montre la structure de la lignine proposée par E. Adler, Wood Sci. Technol. 1977, 11, page 169.

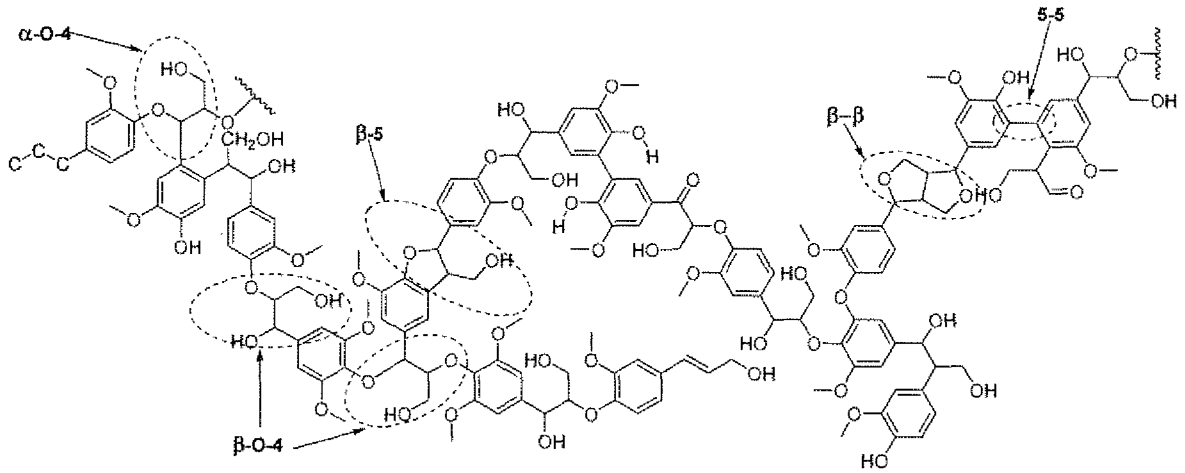

Figure 2

Les liaisons éthers représentent environ les deux tiers des liaisons. Plus spécifiquement, les liaisons de type β-O-4 et α-O-4, qui font partie des alkylaryle éthers, sont les plus abondantes. Typiquement, la lignine des angiospermes (bois dur ou hardwood en anglais) contient 60% de liaisons de type β-O-4 et 6-8% de type α-O-4, et la lignine issue des gymnospermes (bois tendre ou softwood en anglais) contient 46% de liaisons de type β-O-4 et 6-8% de type α-O-4. Bien que la proportion de ces liaisons varie considérablement d'une espèce à une autre, des valeurs typiques extraites de M. P. Pandey, C. S. Kim, Chem. Eng. Technol,, 2011, 34, 29, ont été répertoriées dans le tableau de la Figure 3.

| Liaison | Bois tendre ou Softwood (épicéa) % | Bois dur ou Hardwood (bouleau) % |
|---|---|---|
| **β-O-4** | 46 | 60 |
| **α-O-4** | 6-8 | 6-8 |
| **4-O-5** | 3,5-4 | 6,5 |
| **β-5** | 9-12 | 6 |
| **5-5** | 9,5-11 | 4,5 |
| **β-1** | 7 | 7 |
| **β-β** | 2 | 3 |
| **Autres** | 13 | 5 |

Figure 3

Les structures chimiques des types de liaisons les plus abondantes présentes dans la lignine, sont représentées en Figure 4.

Les types de liaisons les plus abondantes dans la lignine

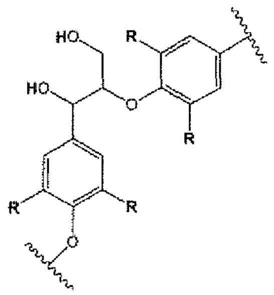

β–O–4

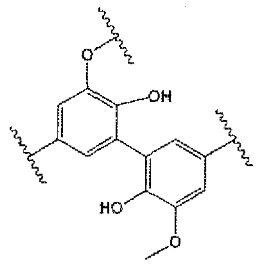

5–5

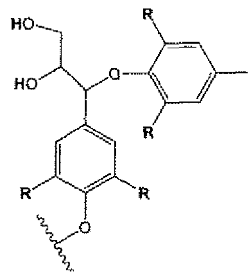

α–O–4

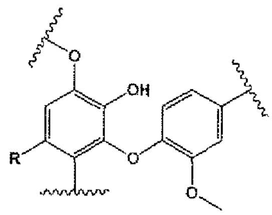

4–O–5

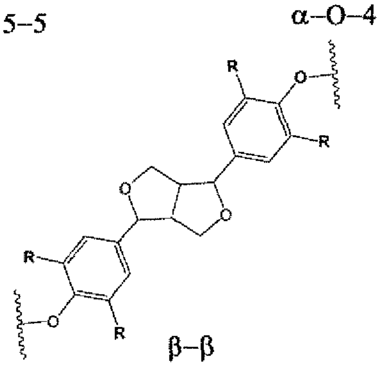

β–β

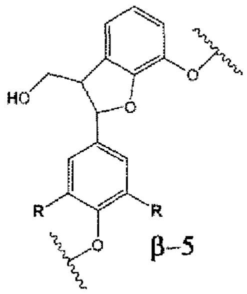

β–5

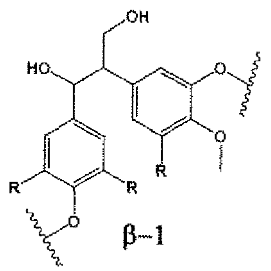

β–1

Figure 4

La lignine représente le plus grand réservoir renouvelable de composés aromatiques disponibles. En raison de sa forte teneur en aromatiques, la lignine a un grand potentiel pour fonctionner comme une alternative aux ressources fossiles non renouvelables pour la production de produits chimiques aromatiques à haute valeur ajoutée, c'est-à-dire des produits dont la transformation augmente de façon considérable leur valeur commerciale. A titre de produits chimiques aromatiques à haute valeur ajoutée, on peut citer, par exemple, le 4-hydroxy-3-méthoxybenzaldéhyde ou la vanilline, le 4-propylbenzène-1,2-diol ou le 4-(3-hydroxypropyl)-1,2-benzènediol.

Ainsi, la valorisation de la lignine implique sa conversion en produits aromatiques précieux et utiles. A ce jour, peu de produits, en particulier aromatiques, purs ou pouvant être facilement purifiés sont obtenus directement à partir de la lignine compte tenu de la structure très hétérogène de la lignine comprenant plusieurs types de résidus et de liaisons différentes, de la grande difficulté à réaliser un clivage sélectif des liaisons de la lignine, ainsi que la difficulté de purification des mélanges finaux obtenus. En effet, à ce jour, les procédés de production de produits aromatiques à partir de la lignine génèrent un grand nombre de produits ayant des propriétés physiques et chimiques qui se ressemblent. Ainsi leurs séparations notamment en produit pur est très difficile par les techniques conventionnelles de purification (chromatographie ou distillation). Le seul et plus important produit aromatique obtenu à partir de la lignine est le 4-hydroxy-3-méthoxybenzaldéhyde ou la vanilline.

M. B. Hocking, J. Chem. Educ., 1997, 74, pages 1055-1059, a décrit la synthèse de la vanilline à partir des ligninesulfonates (lignine issue du procédé sulfite). Ce procédé présente toutefois quelques inconvénients, notamment, la production de grandes quantités d'effluents de déchets (160 kg de liquides caustiques pour chaque kg produit de vanilline). Vu la prise de conscience croissante du public sur les questions environnementales, les coûts de traitement des effluents non durables sont devenus importants, et par conséquent, les usines utilisant ce procédé de synthèse de la vanilline ont commencé à fermer.

Depuis 1993, la société norvégienne Borreegaard constitue le seul producteur de vanilline à partir de la ligninosulfonate. L'étape clé de ce procédé, initialement développé par Monsanto, est la réaction d'oxydation qui se réalise à l'aide d'un catalyseur à base de cuivre, lequel catalyseur étant par la suite recyclé. Cette étape est illustrée dans la Figure 5.

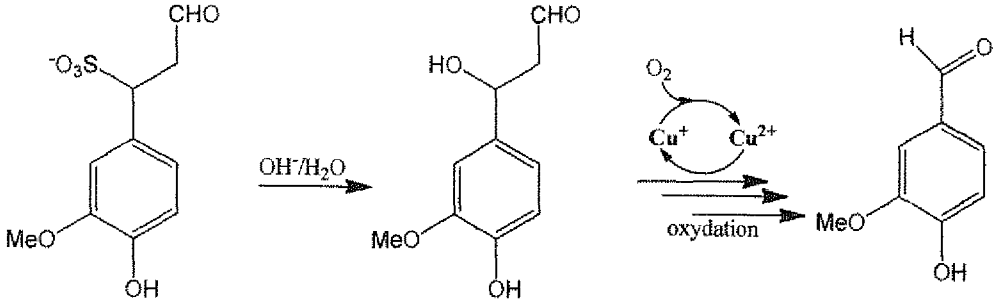


Figure 5

Ce procédé utilise une technologie d'ultrafiltration couplé à une technologie d'osmose inverse, qui permet la réduction du volume des flux de déchets ainsi que l'augmentation du rendement en vanilline (US 4,151,207). Ce procédé permet à partir de 1000 kg de bois, d'obtenir : 400 kg (40 %) de cellulose de spécialités, 400 kg (40 %) de lignine, 3 kg (0,3 %) de vanilline, 20 kg (2 %) de levure, 50 (5 %) kg d'éthanol, et 45 kg (4,5 %) de $CO_2$, avec une valorisation de la chaleur générée par le procédé comme décrit par F. G. Calvo-Flores and J. A. Dobado, ChemSusChem., 2010, 3, pages 1227 - 1235. Le rendement en vanilline par ce procédé est très faible en vanilline (inférieur à 1%). Par ailleurs, ce procédé permet la synthèse de la vanilline seule étant donné qu'il se base essentiellement sur la présence des résidus G. Les molécules aromatiques issues des autres résidus n'ont pas été valorisées par ce procédé.

Zhang et al décrivent un procédé de dépolymérisation de la lignine de bois dur et de la lignine de bois mou , par action de silanes et de boranes. Ce document décrit l'obtention de molécules ayant une masse molaire moyenne en poids égale à 465 g/mol. ( J. Zhang, Y. Chen, M.A.Brook, Acs sustanainable Chemistry and engineering, 2014,2,1983-1991)

La valorisation de la lignine impliquant sa conversion en produits aromatiques précieux et utiles continue à susciter beaucoup d'intérêt. Le problème majeur des chimistes est de développer, à partir de la lignine, des procédés :

- pouvant faire face aux caractéristiques variables de la structure de la lignine, et
- qui permettent de produire des composés chimiques aromatiques traditionnellement obtenus par des méthodes pétrochimiques, avec une bonne pureté ou pouvant être facilement purifiés, et qui pourront servir comme composés de base dans la fabrication de combustibles, de composants électroniques, de polymères plastiques, de caoutchouc, de médicaments, de vitamines, de produits cosmétiques, de parfums, de produits alimentaires, de fils et fibres synthétiques, de cuirs synthétiques, de colles, de pesticides, d'engrais, etc.

Il existe donc un réel besoin d'un procédé permettant la préparation d'un composé aromatique, de bonne pureté (au moins 90% en masse, par rapport à la masse totale des composés aromatiques obtenus) ou pouvant facilement être purifié, à partir de la lignine et palliant les inconvénients de l'art antérieur.

En particulier, il existe un réel besoin d'un procédé permettant la préparation d'un composé aromatique à partir de la lignine, ledit procédé :

- permettant de préparer des composés aromatiques, en particulier des composés aromatiques monocycliques, avec une bonne pureté (au moins 90 % en masse, par rapport à la masse totale des composés aromatiques obtenus) ou pouvant être facilement purifiés ;
- présentant une sélectivité qui puisse être adaptée en fonction des composés aromatiques que l'on cherche à préparer ;
- pouvant s'adapter à la versatilité et l'hétérogénéité de la lignine, qui dépendent du type de végétation dont provient la lignine ainsi que de son procédé d'extraction ;
- présentant une grande productivité qui se traduit par une grande conversion de la lignine en composés aromatiques de bonnes puretés ou pouvant être facilement purifiés ;
- pouvant être mis en œuvre dans des conditions opératoires douces et industriellement intéressantes ; et/ou
- étant simple à mettre en œuvre.

Le développement d'un procédé de dépolymérisation convergente pourra donc contribuer d'une façon significative à la valorisation de la lignine par sa conversion en produits aromatiques utiles tout en répondant aux exigences précitées. Un procédé de dépolymérisation convergente de la lignine a pour principe de conduire à la réduction de la lignine en petits oligomères qui diffèrent par leur degré d'oxygénation. Ces derniers sont réduits (désoxygénés) au fur et à mesure pour converger vers un seul produit qui s'accumule dans le milieu réactionnel. Or, la dépolymérisation de la lignine est difficile et représente un défi, car sa structure est hautement fonctionnalisée et ramifiée et son encombrement stérique peut limiter l'accès du catalyseur aux sites actifs. D'autre part, l'hétérogénéité chimique de la lignine qui est due à la présence de plusieurs résidus G, H, S présents à des taux variables selon la source végétale, et à la présence de divers types de liaisons C-O et C-C de type aryle éther, aryle glycérol et β-aryle éther, complique l'obtention de produits chimiques purs lors de la transformation de la lignine.

Compte tenu de la difficulté à procéder à une dépolymérisation directe de la lignine, les scientifiques ont synthétisé des modèles, chimiquement purs, représentatifs des liaisons éthers présentes dans la lignine, pour en étudier la réactivité (J. Zakzeski, P. C. A. Bruijnincx, A. L. Jongerius and B. M. Weckhuysen, Chem Rev., 2010, 110, page 3552). La majorité des études visant la dépolymérisation de la lignine s'est focalisée sur ces modèles et n'a pas pu être mise en œuvre sur la structure complexe de lignines naturelles. Divers complexes organométalliques ont été utilisés pour catalyser les réactions réalisées sur les modèles des liaisons éthers dans la lignine, comme par exemple :

- le ruthenium (a) J. M. Nichols, L. M. Bishop, R. G. Bergman, J. A. Ellman, J. Am. Chem. Soc, 2010, 132, pages 12554-12555 ; b) A. Wu, B. O. Patrick, E. Chung and B. R. James, Dalton Trans., 2012, 41, page 11093 ; c) T. vom Stein,T. Weigand, C. Merkens, Jurgen Klankermayer, W. Leitner, ChemCatChem, 2013, 5, pages 439-441),
- le vanadium (S. Son and F. D. Toste, Angew. Chem. Int. Ed. 2010, 49, pages 3791-3794), et
- le nickel (A. G. Sergeev and J. F. Hartwig, Science, 2011, 332, page 439).

Etant donné la structure polymérique complexe, hétérogène et fortement encombrée de la lignine qui complique sa dépolymérisation, les procédés de dépolymérisation développés dans la littérature se réalisent, en général, dans des conditions drastiques de température et de pression et mettent en œuvre des métaux en quantités catalytiques élevées. D'autre part, rares sont les procédés qui ont pu être extrapolés des modèles chimiquement purs à la dépolymérisation de la lignine. En 2013, la première réduction organocatalytique des composés modèles de la lignine a été décrite par Feghali et Cantat (E. Feghali, T. Cantat, Chem. Commun,, 2014, 50, pages 862-865). Ces derniers ont montré que le $B(C_6F_5)_3$ est un catalyseur d'hydrosilylation efficace et sélectif pour le clivage réducteur des liaisons alkylaryle éthers et, en particulier, des modèles de motifs α-O-4 et β-O-4. De plus, la réduction a été réalisée dans des conditions douces (température ambiante de 2 à 16 heures), et a pu être menée avec une source d'hydrure stable à l'air et peu coûteuse comme le polyméthylhydrosiloxane (PMHS) et le tétraméthyldisilazane (TMDS). Néanmoins, ce procédé n'a pu être extrapolé à la dépolymérisation de la lignine.

A ce jour, il n'existe pas de procédé de dépolymerisation réductrice de la lignine pour la préparation de composés aromatiques. Les seuls procédés qui décrivent la dépolymérisation de la lignine en catalyse homogène sont ceux réalisés décrits par Toste *et al.* et Ragauskas *et al.*

- Le procédé de Toste *et al.* qui a été développé sur les modèles de liaisons C-O du motif β-O-4 de la lignine (S. Son and F. D. Toste, Angew. Chem. Int. Ed. 2010, 49, pages 3791-3794) a pu être extrapolé sur la lignine extraite de *Miscanthus giganteus* ou herbe aux éléphants (J. M. W. Chan, S. Bauer, H. Sorek, S. Sreekumar, K. Wang, F. D. Toste, ACS Catal, 2013, 3, pages 1369-1377). Dans ce procédé, un catalyseur de vanadium a été utilisé par Toste *et al.* pour le clivage de liaisons C-O du motif β-O-4 de la lignine et la formation d'aryle énones. Cette transformation redox est effectuée dans l'acétate d'éthyle à 80°C pendant 24 h. La charge de catalyseur est de 10% en masse. Les résultats des études CPG et RMN 2D de la dépolymérisation de la lignine dioxasolv et acetosolv ressemblaient aux données obtenues avec les modèles de lignine, ce qui confirme la sélectivité pour les liaisons β-O-4. Enfin, les auteurs ont pu identifier et quantifier par GC/MS, des composés phénoliques volatils (comme la vanilline, l'acide vanillique, l'acide syringique et le syringaldéhyde) produits dans la réaction. Néanmoins, aucun produit chimique n'a pu être isolé pur à partir de ce procédé et des mélanges complexes partiellement caractérisés ont été obtenus avec des rendements faibles pour chaque produit. En effet, dans le cas de Toste *et al.,* la dépolymérisation se réalise sur des herbes qui sont déjà formés d'un mélange des trois résidus H, G et S, le procédé mis en œuvre est un procédé redox qui, au lieu de converger les produits obtenus vers un seul produit, garde la même diversité en produits obtenus que celle présente dans la lignine de départ.
- Les groupes de Toste, Ellman et Hartwig ont regroupé leurs résultats sur la réduction de la lignine et de ses modèles en catalyse homogène dans la demande internationale WO2011003029. Les précurseurs utilisés sont des dérivés de vanadium, de ruthénium et de rhodium. Seuls les complexes à base de vanadium et de ruthénium ont été utilisés pour la dépolymérisation redox de la lignine extraite de *Miscanthus giganteus.* Néanmoins, aucun produit chimique pur n'a pu être isolé ni identifié par ce procédé et des mélanges partiellement caractérisés ont été obtenus.
- En 2009, Ragauskas *et al.* (M. Nagy, K. David, G. J. P. Britovsek and A. J. Ragauskas, Holzforschung, 2009, 63,

page 513) ont réussi à dépolymériser la lignine éthanol organosolv (EOL) (soluble dans l'éthanol) issue du pin dans des conditions réductrices. Dans cette étude, des catalyseurs hétérogènes classiques ainsi que des nouveaux catalyseurs homogènes ont été employés pour le clivage de liaison diaryle éthers et dialkyle éthers. En employant les conditions d'hydrogénolyse : 5 MPa $H_2$ ; 175°C ; 20 heures, le catalyseur de ruthénium augmente efficacement la solubilité de la lignine (solubilité jusqu'à 96%) et contribue à sa dégradation. Une diminution de l'ordre de 10% à 20% de la masse molaire moyenne en masse (Mw) a été obtenue (Mw =1900-2100 g/mol), ce qui correspond à un dégrée de polymérisation (DP) de 10 à 11 unités de monomères (L.B. Davin, L.B., N. G. Lewis, Curr, Opin. Biotechnol., 2005, 16, pages 407 - 415). D'autre part, d'après les auteurs, l'hydrogénolyse des groupements diaryles éthers et alkylaryles éthers est accompagnée d'une réaction d'hydrogénation simultanée du cycle aromatique et il n'est ainsi pas possible d'isoler de composés aromatiques. Enfin, l'identification ainsi que la formation détaillée des produits de réaction et des voies de clivage n'ont pas été élucidés étant donné que les produits obtenus sont des oligomères dont la structure est très difficilement identifiable. Ragauskas *et al.* ne mentionne et n'obtient pas de molécules de faibles masses molaires.

Comme déjà indiqué, en raison de sa forte teneur en composés aromatiques, la lignine a un grand potentiel pour fonctionner comme une alternative aux ressources fossiles non renouvelables pour la production de produits chimiques aromatiques à haute valeur ajoutée. Toutefois, en raison de sa structure amorphe, très diversifiée en termes de résidus contenus, polymérique basée sur des liaisons éthers fortes, et contenant un grand nombre de liaisons de différents types, sa dépolymérisation pour produire sélectivement des molécules utilisables représente un défi (P. J. Deuss, K. Barta and J. G. de Vries, Cata/. Sci. Technol., 2014, Accepted Manuscript ; DOI: 10.1039/C3CY01058A). En outre, les lignines sont structurellement très diversifiées et, en fonction de la source végétale utilisée, elles peuvent contenir des proportions différentes des trois monomères de base que sont l'alcool p-coumaryle, l'alcool coniféryle et l'alcool sinapyle.

Il existe donc un réel besoin d'un procédé permettant la préparation d'un composé aromatique à partir de la lignine pouvant mettre en œuvre une étape de dépolymérisation de la lignine palliant les inconvénients de l'art antérieur.

En particulier, il existe un réel besoin d'un procédé permettant la préparation d'un composé aromatique à partir de la lignine, ledit procédé :

- permettant de préparer de la vanilline mais également d'autres composés aromatiques, en particulier des composés aromatiques monocycliques, avec une bonne pureté (au moins 90% en masse du composé aromatique monocyclique désiré, par rapport à la masse totale des composés aromatiques monocycliques obtenus) ou pouvant être facilement purifiés ;
- présentant une grande productivité qui se traduit par une grande conversion de la lignine en composés aromatiques de bonnes puretés ou pouvant être facilement purifiés ;
- présentant une sélectivité modulable par rapport au(x) composé(s) aromatique(s) que l'on cherche à préparer ;
- pouvant être mis en œuvre dans des conditions opératoires douces et industriellement intéressantes ;
- mettant en œuvre une étape de dépolymérisation de la lignine :
    - simple à mettre en œuvre ;
    - qui peut se réaliser dans des conditions opératoires douces et industriellement intéressantes ;
    - qui peut s'adapter à la versatilité et à l'hétérogénéité de la lignine qui sont des caractéristiques dépendant du type de végétation dont provient la lignine et de son procédé d'extraction ;
    - permettant un clivage sélectif de certaines liaisons de la lignine.

La présente invention a précisément pour but de répondre à ces besoins en fournissant un procédé de préparation de composés aromatiques monocycliques de formule (I)

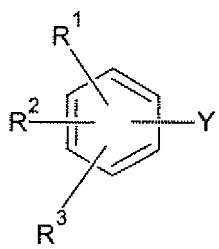

(I)

dans laquelle

- $R^1$, $R^2$, $R^3$, représentent, indépendamment l'un de l'autre, un atome d'hydrogène, un groupe hydroxyle, un groupe alkoxy, un groupe siloxy ;
- Y représente un groupe alkyle, un groupe alcényle, un groupe alcynyle, un groupe carbonyle $-CR^4=O$ avec $R^4$ représentant un atome d'hydrogène, un groupe alkyle, un groupe hydroxyle, un groupe alkoxy ;

lesdits groupes alkyle, alcényle et alcynyle étant éventuellement substitués ;
caractérisé en ce qu'il comprend une étape de dépolymérisation de la lignine par clivage sélectif de la liaison carbone $sp^3$-oxygène des alkylaryles éthers du type β-O-4, α-O-4, β-5, β-1, β-β présents dans la lignine, ladite étape de dépolymérisation comprenant la réaction,

- d'une lignine contenant un taux de soufre inférieur à 1,5% en masse, par rapport à la masse totale de la lignine, avec
- un composé silane de formule (II)

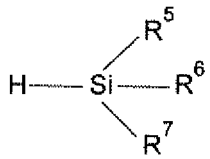

(III)

dans laquelle

- $R^5$, $R^6$ et $R^7$ représentent, indépendamment l'un de l'autre, un atome d'hydrogène, un atome d'halogène, un groupe hydroxyle, un groupe alkyle, un groupe alcényle, un groupe alcynyle, un groupe alkoxy, un groupe aryloxy, un groupe silylé, un groupe siloxy, un groupe aryle, un groupe amino, lesdits groupes alkyle, alcényle, alcynyle, alkoxy, silylé, siloxy, aryle et amino étant éventuellement substitués, ou
- $R^7$ est tel que défini ci-dessus et $R^5$ et $R^6$, pris ensemble avec l'atome de silicium auquel ils sont liés forment un hétérocycle silylé éventuellement substitué ;

en présence d'un catalyseur. ; et en ce que les composés aromatiques monocycliques de formule (I) ont une masse molaire moyenne en poids inférieure à 1500g/mol sous forme silylée et une masse molaire moyenne en poids inférieure à inférieure 400 g/mol sous forme non silylée ; et en ce que la lignine est extraite par les procédés organosolv.

Le procédé de l'invention comprend donc une étape de dépolymérisation de la lignine. La dépolymérisation de la lignine permet un clivage sélectif des liaisons carbone $sp^3$-oxygène des alkylaryle éthers présents dans la lignine et vise ainsi essentiellement les liaisons carbone $sp^3$-oxygène du type β-O-4, α-O-4, β-5, β-1, β-β. Sans vouloir être lié par la théorie, le clivage des liaisons carbone $sp^3$-oxygène au niveau des liaisons du type β-O-4, α-O-4 et β-β conduit à la fois à la dépolymérisation de la lignine et à la modification de sa structure, alors que le clivage des liaisons carbone $sp^3$-oxygène au niveau des liaisons du type β-5 et β-1, conduit à la modification de la structure de la lignine sans la coupure de la liaison entre deux monomères successifs.

Les liaisons carbone $sp^2$-oxygène des aryles éthers présents dans la lignine (essentiellement les liaisons du type 5-5 et 4-O-5) ainsi que toute autre liaison carbone $sp^2$-oxygène présente dans la lignine restent intactes lors du procédé de l'invention.

L'étape de dépolymérisation de la lignine dans le procédé de l'invention est réalisée dans des conditions opératoires douces et permet de s'affranchir des conditions réactionnelles drastiques de température et de pression utilisées traditionnellement dans la littérature pour la dépolymérisation de la lignine. La dépolymérisation de la lignine dans le procédé de l'invention est également industriellement intéressante car elle permet de réduire des coûts par l'utilisation des silanes de formule (III) qui sont stables à l'air et peu coûteux.

Par ailleurs, la dépolymérisation de la lignine peut générer du méthane et de l'hydrogène (de l'ordre de 7 à 20 % en masse de la masse totale de la lignine introduite). Ces deux gaz peuvent éventuellement être utilisés comme combustibles pour fournir de l'énergie au procédé de l'invention.

Dans l'étape de dépolymérisation, les composés silanes de formule (III) assurent le clivage par réduction des liaisons carbone $sp^3$-oxygène des alkylaryle éthers présents dans la lignine, en conditions catalytiques.

Le procédé de l'invention permet la préparation des composés aromatiques monocycliques de formule (I) avec une bonne pureté, c'est-à-dire, une pureté supérieure ou égale à 90 % en masse, de préférence comprise entre 90 et 99,9 % en masse, par rapport à la masse totale des composés aromatiques monocycliques de formule (I).

Avec le procédé de l'invention, les composés aromatiques monocycliques de formule (I) peuvent également

être obtenus en mélange avec des oligomères formés par les entités monomériques liées entre elles par les liaisons qui ne peuvent pas être clivées par le procédé de l'invention, notamment les liaisons C-C et les liaisons diaryléther. Dans ce cas, les composés aromatiques monocycliques de formule (I) peuvent être facilement purifiés par des techniques de séparation bien connues de l'homme du métier et classiquement utilisées dans ce domaine, comme par exemple, la chromatographie sur colonne, la distillation, etc.

En effet, contrairement aux procédés décrits dans la littérature, dans le procédé de l'invention, tous les produits volatils susceptibles d'être formés continuent à subir des réactions de réduction pour finir par donner un seul produit volatil (principe de la convergence), ce qui facilite leur purification par distillation. De plus, compte tenu du fait que les produits obtenus sont en général silylés, la température d'ébullition du produit est diminuée ce qui facilite les conditions opératoires de distillation. Par ailleurs, comme les produits secondaires sont des oligomères non volatils, ils sont facilement séparables du produit aromatique monocyclique de formule (I) désiré, par distillation.

Il est à noter que les composés aromatiques monocycliques de formule (I) obtenus par le procédé de l'invention, peuvent également être purifiés par la chromatographie sur colonne.

Les composés aromatiques monocycliques de formule (I) ont en général une masse molaire moyenne en poids inférieure à 2000 g/mol, de préférence inférieure à 1500g/mol sous forme silylée (c'est-à-dire lorsque tous les atomes d'oxygène -OH de ces composés-OH sont sous forme silylée -O-Si). Après désilylation par hydrolyse par exemple, toutes les liaisons -O-Si sont clivées et la masse molaire moyenne en poids des composés aromatiques monocycliques de formule (I) sous forme non silylée ainsi obtenu est inférieure à 400 g/mol. La masse molaire moyenne en poids des composés obtenus peut être déterminée par toute méthode connue de l'homme du métier notamment par la chromatographie d'exclusion stérique (SEC pour Size Exclusion Chromatography en anglais).

Par ailleurs, les composés aromatiques monocycliques de formule (I) sont obtenus avec un bon rendement (de l'ordre de 10 à 70 % en masse par rapport à la masse de la lignine départ, par exemple).

Dans le cadre de la présente invention, le rendement est défini comme étant le rapport entre la masse de composés aromatiques isolés de formule (I), ayant une masse molaire moyenne en poids inférieure à 2000 g/mol, de préférence inférieure à 1500 g/mol pour les composés sous forme silylée ou inférieure 400g/mo1 pour les composés sous forme non silylée, et la masse de la lignine introduite initialement :

$$\text{Rendement} = m(\text{composés aromatiques de formule (I)}) / m(\text{lignine})$$

m étant la masse de la lignine initialement introduite.

Il est à noter que la détermination de la quantité exacte de la lignine n'est pas aisée. Ragauskas *et al.* (M. Nagy, K. David, G. J. P. Britovsek and A. J. Ragauskas, Holzforschung, 2009, 63, 513) décrit une approximation de cette quantité qui associe à chaque motif une masse molaire d'environ 200 g/mol. Vu la complexité et la diversité des structures des lignines qui sont formées, de proportions différentes des résidus H, G et S présents dans les lignines, et étant donnée la présence de plusieurs sortes d'impuretés ayant des structures non connues, il est très difficile de déterminer une formule moléculaire bien définie pour décrire la lignine. Par conséquent, la détermination de rendement molaire dans le cas de la lignine est impossible, les rendements sont exprimés en masse de produit obtenu par rapport à la masse de lignine initialement ajoutée.

On entend par « alkyle », au sens de la présente invention, un radical carboné linéaire, ramifié ou cyclique, saturé, éventuellement substitué, comprenant 1 à 12 atomes de carbone, de préférence 1 à 8 atomes de carbone. A titre d'alkyle saturé, linéaire ou ramifié, on peut citer par exemple les radicaux méthyle, éthyle, propyle, butyle, pentyle, hexyle, octyle, nonyle, décyle, undécyle, dodécanyle et leurs isomères ramifiés. Comme alkyle cylique, on peut citer les radicaux cyclopropyle, cyclobutyle, cyclopentyle, cyclohexyle, bicylco[2,1,1] hexyle, bicyclo[2,2,1] heptyle.

Par « alcényle » ou « alcynyle », on entend un radical carboné insaturé linéaire, ramifié ou cyclique, éventuellement substitué, ledit radical carboné insaturé comprenant 2 à 12 atomes de carbone, de préférence 2 à 8 atomes de carbone, comprenant au moins une double (alcényle) ou une triple liaison (alcynyle). A ce titre, on peut citer, par exemple, les radicaux éthylényle, propylényle, butényle, pentényle, hexényle, acétylényle, propynyle, butynyle, pentynyle, hexynyle et leurs isomères ramifiés. Comme alkyles cycliques insaturés, on peut citer par exemple le cyclopentényle, le cyclohexényle.

Le groupe alkyle, alcényle et alcynyle, au sens de l'invention, peuvent être éventuellement substitués par un ou plusieurs groupes hydroxyle ; un ou plusieurs groupes alkoxy ; un ou plusieurs groupes siloxy ; un ou plusieurs atomes d'halogène choisis parmi les atomes de fluor, chlore, brome et iode ; un ou plusieurs groupes nitro ($-NO_2$) ; un ou plusieurs groupes nitrile (-CN) ; un ou plusieurs groupes aryle, avec les groupes alkoxy et aryle tels que définis dans le cadre de la présente invention.

Le terme « aryle » désigne de manière générale un substituant aromatique cyclique comportant de 6 à 20, de préférence de 6 à 12 atomes de carbone. Dans le cadre de l'invention le groupe aryle peut être mono- ou polycyclique. A titre indicatif, on peut citer les groupes phényle, benzyle et naphtyle. Le groupe aryle peut être éventuellement substitué

par un ou plusieurs groupes hydroxyle, un ou plusieurs groupes alkoxy, un ou plusieurs groupes «siloxy», un ou plusieurs atomes d'halogène choisis parmi les atomes de fluor, chlore, brome et iode, un ou plusieurs groupes nitro (-$NO_2$), un ou plusieurs groupes nitrile (-CN), un ou plusieurs groupes alkyle, avec les groupes alkoxy et alkyle tels que définis dans le cadre de la présente invention.

Le terme « hétéroaryle » désigne de manière générale un substituant aromatique mono- ou polycyclique comportant de 5 à 10 membres, de préférence de 5 à 7 membres dont au moins 2 atomes de carbone, et au moins un hétéroatome choisi parmi l'azote, l'oxygène, le bore, le silicium, le phosphore et le soufre. Le groupe hétéroaryle peut être mono- ou poly-cyclique. A titre indicatif, on peut citer les groupes furyle, benzofuranyle, pyrrolyle, indolyle, isoindolyle, azaindolyle, thiophényle, benzothiophényle, pyridyle, quinolinyle, isoquinolyle, imidazolyle, benzimidazolyle, pyrazolyle, oxazolyle, isoxazolyle, benzoxazolyle, thiazolyle, benzothiazolyle, isothiazolyle, pyridazinyle, pyrimidilyle, pyrazinyle, triazinyle, cinnolinyle, phtalazinyle, quinazolinyle. Le groupe hétéroaryle peut être éventuellement substitué par un ou plusieurs groupes hydroxyle, un ou plusieurs groupes alkoxy, un ou plusieurs atomes d'halogène choisis parmi les atomes de fluor, chlore, brome et iode, un ou plusieurs groupes nitro (-$NO_2$), un ou plusieurs groupes nitrile (-CN), un ou plusieurs groupes aryle, un ou plusieurs groupes alkyle, avec les groupes alkyle, alkoxy et aryle tels que définis dans le cadre de la présente invention.

Le terme « alkoxy » signifie un groupe alkyle, alcényle et alcynyle, tels que définis ci-dessus, lié par un atome d'oxygène (-O-alkyle, O-alcényle, O-alcynyle).

Le terme « aryloxy » signifie un groupe aryle tel que défini ci-dessus, lié par un atome d'oxygène (-O-aryle).

Le terme « hétérocycle » désigne de manière générale un substituant mono- ou polycyclique, comportant de 5 à 10 membres, de préférence de 5 à 7 membres, saturé ou instauré, contenant de 1 à 4 hétéroatomes choisis indépendamment l'un de l'autre, parmi l'azote, l'oxygène, le bore, le silicium, le phosphore et le soufre. A titre indicatif, on peut citer les substituants morpholinyle, pipéridinyle, pipérazinyle, pyrrolidinyle, imidazolidinyle, imidazolinyle, pyrazolidinyle, tétrahydrofuranyle, tétrahydropyranyle, thianyle, oxazolidinyle, isoxazolidinyle, thiazolidinyle, isothiazolidinyle. L'hétérocycle peut être éventuellement substitué par un ou plusieurs groupes hydroxyle, un ou plusieurs groupes alkoxy, un ou plusieurs groupes aryle, un ou plusieurs atomes d'halogène choisis parmi les atomes de fluor, chlore, brome et iode, un ou plusieurs groupes nitro (-$NO_2$), un ou plusieurs groupes nitrile (-CN), un ou plusieurs groupes alkyle, avec les groupes alkyle, alkoxy et aryle tels que définis dans le cadre de la présente invention.

Par atome d'halogène, on entend un atome choisi parmi les atomes de fluor, chlore, brome et iode.

Par groupe « silylé », on entend un groupe de formule [-$Si(X)_3$] dans lequel chaque X, indépendamment l'un de l'autre, est choisi parmi un atome d'hydrogène ; un ou plusieurs atomes d'halogène choisis parmi les atomes de fluor, chlore, brome ou iode ; un ou plusieurs groupes alkyle ; un ou plusieurs groupes alkoxy ; un ou plusieurs groupes aryle ; un ou plusieurs groupes siloxy ; avec les groupes alkyle, alkoxy, aryle et siloxy tels que définis dans le cadre de la présente invention. Lorsque l'un au moins des X représente plusieurs groupes siloxy, lesdits groupes siloxy peuvent se répéter plusieurs fois de manière à conduire à des organosilanes polymériques de formule générale

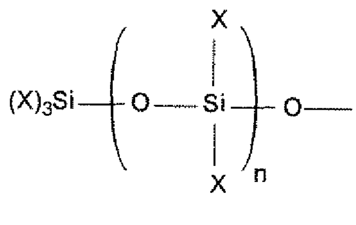

dans laquelle X est tel que défini ci-dessus et n est un nombre entier compris entre 1 et 20000, avantageusement entre 1 et 5000, plus avantageusement entre 1 et 1000. A ce titre on peut citer, par exemple, le polydiméthylsiloxane (PDMS), le polyméthylhydroxysiloxane (PMHS) et le tétraméthyldisiloxane (TMDS).

Par groupe « siloxy », on entend un groupe silylé, tel que défini ci-dessus, lié par un atome d'oxygène (-O-$Si(X)_3$) avec X tel que défini ci-dessus.

Au sens de l'invention, par « hétérocycle silylé », on entend un substituant mono-ou polycyclique, comportant de 5 à 15 membres, de préférence de 5 à 7 membres, saturé ou instauré, contenant au moins un atome de silicium et éventuellement au moins un autre hétéroatome choisi parmi l'azote, l'oxygène et le soufre. Ledit hétérocycle silylé peut être éventuellement substitué par un ou plusieurs groupes hydroxyle ; un ou plusieurs groupes alkyle, un ou plusieurs groupes alkoxy ; un ou plusieurs atomes d'halogène choisis parmi les atomes de fluor, chlore, brome et iode ; un ou plusieurs groupes aryle, avec les groupes alkyle, alkoxy et aryle tels que définis dans le cadre de la présente invention. Parmi les hétérocycles silylés, on peut citer par exemple, le 1-silacyclo-3-pentène ou le 1-méthyl-1,1-dihydrido-2,3,4,5-tétraphényl-1-silacyclopentadiène, suivant les formules de la Figure 6.

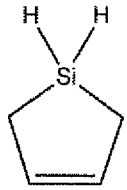 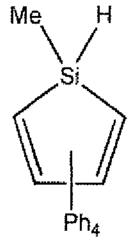

1-silacyclo-3-pentène 1-méthyl-1-hydrido-2,3,4,5-tétraphényl-1-silacyclopentadiène

Figure 6

On peut encore citer, par exemple, le méthyl siloxane, le 1-phényl-1-silacyclohexane, le 1-sila-bicyclo[2.2.1]heptane, le 1-méthyl-1-silacyclopentane, le 9,9-dihydro-5-silafluorène répondant aux formules de la Figure 7.

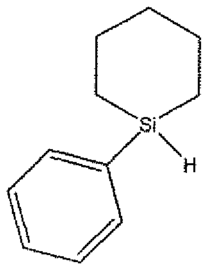 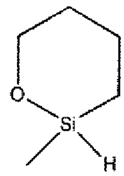

methyl siloxane

1-phenyl-1-silacyclohexane

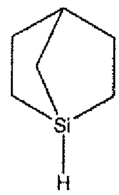

1-sila-bicyclo[2.2.1]heptane

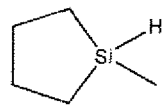 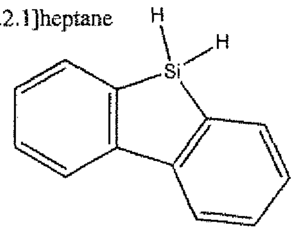

1-methyl-1-silacyclopentane 9,9-dihydro-5-silafluorene

Figure 7

Par polyol on entend : un composé organique caractérisé par la présence d'un certain nombre de groupements hydroxyle (-OH). Dans le cadre de cette invention un composé polyol contient au moins un groupement hydroxyle. Pour ce faire, on entend par polyol un composé de formule Z-$(OH)_n$, dans lequel n est supérieur ou égale à 1, et Z est choisi parmi un ou plusieurs groupes alkyle, un ou plusieurs groupes alkoxy, un ou plusieurs groupes siloxy, un ou plusieurs groupes aryle, un ou plusieurs groupes hétéroaryle avec les groupes alkyle, alkoxy et aryle tels que définis dans le cadre de la présente invention.

Par groupe « amino », on entend un groupe de formule -$NR^8R^9$, dans laquelle :

- $R^8$ et $R^9$ représentent, indépendamment l'un de l'autre, un atome d'hydrogène, un groupe alkyle, un groupe alcényle, un groupe alcynyle, un groupe aryle, un groupe hétéroaryle, un hétérocycle, un groupe silylé, un groupe siloxy, avec les groupes alkyle, alcényle, alcynyle, aryle, hétéroaryle, hétérocycle, silylé, siloxy, tels que définis dans le cadre de la présente invention ; ou

- $R^8$ et $R^9$, pris ensemble avec l'atome d'azote auquel ils sont liés, forment un hétérocycle éventuellement substitué par un ou plusieurs groupes hydroxyle ; un ou plusieurs groupes alkyle ; un ou plusieurs groupes alkoxy ; un ou plusieurs atomes d'halogène choisis parmi les atomes de fluor, chlore, brome et iode ; un ou plusieurs groupes nitro ($-NO_2$) ; un ou plusieurs groupes nitrile (-CN) ; un ou plusieurs groupes aryle ; avec les groupes alkyle, alkoxy et aryle tels que définis dans le cadre de la présente invention.

Par « bois » on entend un tissu végétal qui correspond au xylème secondaire chez les plantes. Le terme bois englobe tous les tissus secondaires formant les troncs, branches et racines des plantes ligneuses.

Par Gymnospermes on entend des plantes appartenant au sous-embranchement paraphylétique des Spermaphytes (plantes à graines) et incluant les plantes dont l'ovule est à nu et est porté par des pièces foliaires groupées sur un rameau fertile (le cône). La plupart des Gymnospermes appartienne à la division des conifères (ou pinophytes).

Par Angiospermes on entend des plantes appartenant au sous-embranchement paraphylétique des Spermaphytes (plantes à graines) et incluant les plantes à fleurs, et donc les végétaux qui portent des fruits. Ces plantes constituent la plus grande partie des espèces végétales sur terre avec 250 000 à 300 000 espèces.

Par Pinacées *(Pinaceae)* ou Abiétacées on entend une famille des plantes gymnospermes et plus précisément de la division des conifères qui compte entre 220 et 250 espèces réparties en 11 genres. Ce sont des arbres ou des arbustes, ayant des feuilles persistantes en aiguille ou en écailles ou caduques.

Par lignine on entend un biopolymère présent dans toutes les plantes et principalement dans les plantes vasculaires, les plantes ligneuses, les plantes herbacées et les algues. La lignine constitue un des principaux composants du bois. La lignine est un polyol riche en groupes aryles tels que définis ci-dessus. Elle est issue d'un tissu végétal notamment des feuilles, des tiges herbacées et des tiges ligneuses. En fonction de son procédé d'extraction et de son origine, la lignine peut contenir d'autres groupes chimiques comme par exemple, des alcènes, des alcynes, des alcools primaires secondaires et tertiaires, des cétones, des acides carboxyliques, des acétals, des hémiacétals, des énols, des éthers, des esters, des alcools allyliques, des alcools homoallyliques, des nitriles, des imines, des amines primaires secondaires et tertiaires, des amides, des halogènes, des sulfures, des thiols, des sulfonates, des sulfones, des sulfates, des sulfoxydes.

De préférence, le procédé de l'invention permet de préparer un composé aromatique monocyclique de formule (I) dans laquelle

- $R^1$, $R^2$, $R^3$, représentent, indépendamment l'un de l'autre, un atome d'hydrogène ; un groupe hydroxyle ; un groupe alkoxy dont le groupe alkyle est choisi parmi les groupes méthyle, éthyle, propyle, butyle et ses isomères ramifiés ; un groupe siloxy ($-O-Si(X)_3$) dont chaque X, indépendamment l'un de l'autre, est choisi parmi un atome d'hydrogène, un groupe alkyle choisi parmi les groupes méthyle, éthyle, propyle, un groupe aryle choisi parmi le phényle et le benzyle, un organosilane polymérique de formule générale

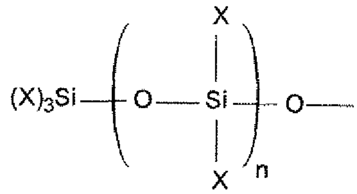

dans laquelle X est tel que défini ci-dessus et n est un nombre entier compris entre 1 et 20000, avantageusement entre 1 et 5000, plus avantageusement entre 1 et 1000 ;

- Y représente un groupe alkyle choisi parmi les groupes méthyle, éthyle, propyle, butyle et ses isomères ramifiés ; un groupe carbonyle $-CR^4=O$ avec $R^4$ représentant un atome d'hydrogène ; un groupe alkyle choisi parmi les groupes méthyle, éthyle, propyle, butyle et ses isomères ramifiés ; un groupe hydroxyle ;

lesdits groupes alkyle étant éventuellement substitués par un ou plusieurs groupes hydroxyle ; un ou plusieurs groupes siloxy ($-O-Si(X)_3$) dont chaque X, indépendamment l'un de l'autre, est choisi parmi un atome d'hydrogène, un groupe alkyle choisi parmi les groupes méthyle, éthyle, propyle, un groupe aryle choisi parmi le phényle et le benzyle, un organosilane polymérique de formule générale

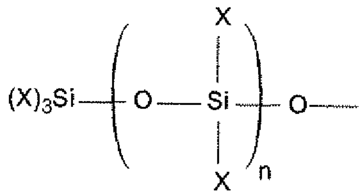

dans laquelle X est tel que défini ci-dessus et n est un nombre entier compris entre 1 et 20000, avantageusement entre 1 et 5000, plus avantageusement entre 1 et 1000.

Plus préférentiellement, le procédé de l'invention permet de préparer un composé aromatique monocyclique de formule (I) dans laquelle

- $R^1$, $R^2$ , $R^3$, représentent, indépendamment l'un de l'autre, un atome d'hydrogène ; un groupe hydroxyle ; un groupe alkoxy dont le groupe alkyle est un groupe méthyle ou un groupe éthyle ; un groupe siloxy (-O-Si$(X)_3$) dont chaque X, indépendamment l'un de l'autre, est un atome d'hydrogène, un groupe méthyle, un groupe éthyle, un groupe phényle, le polydiméthylsiloxane (PDMS), le polyméthylhydroxysiloxane (PMHS), le tétraméthyldisiloxane (TMDS) ;
- Y représente un groupe alkyle choisi parmi les groupes méthyle, éthyle, propyle, butyle et ses isomères ramifiés ; un groupe carbonyle $-CR^4=O$ avec $R^4$ représentant un atome d'hydrogène ; un groupe alkyle choisi parmi les groupes méthyle, éthyle, propyle, butyle et ses isomères ramifiés ; un groupe hydroxyle ;

lesdits groupes alkyle étant éventuellement substitués par un ou plusieurs groupes hydroxyle ; un ou plusieurs groupes siloxy (-O-Si$(X)_3$) dont chaque X, indépendamment l'un de l'autre, est choisi parmi un atome d'hydrogène, un groupe méthyle, un groupe éthyle, un groupe phényle, le polydiméthylsiloxane (PDMS), le polyméthylhydroxysiloxane (PMHS), le tétraméthyldisiloxane (TMDS).

De préférence, l'étape de dépolymérisation, met en œuvre un composé silane de formule (III) dans laquelle $R^5$, $R^6$ et $R^7$ représentent, indépendamment l'un de l'autre, un atome d'hydrogène ; un groupe alkyle choisi parmi les groupes méthyle, éthyle, propyle, butyle et ses isomères ramifiés ; un groupe siloxy (-O-Si$(X)_3$) dont chaque X, indépendamment l'un de l'autre, est un groupe alkyle choisi parmi les groupes méthyle, éthyle, propyle, butyle et ses isomères ramifiés ; un groupe alkoxy dont le groupe alkyle est choisi parmi les groupes méthyle, éthyle, propyle, butyle et ses isomères ramifiés ; un groupe aryle choisi parmi les groupes phényle et benzyle ; lesdits groupes alkyle et aryle étant éventuellement substitués.

Plus préférentiellement, dans le composé silane de formule (III), $R^5$, $R^6$ et $R^7$ représentent, indépendamment l'un de l'autre, un atome d'hydrogène ; un groupe alkyle choisi parmi les groupes méthyle, éthyle, propyle ; un groupe aryle choisi parmi les groupes benzyle et phényle.

Dans le procédé de l'invention, il est essentiel que la lignine utilisée dans l'étape de dépolymérisation soit exempte de soufre, c'est-à-dire qu'elle contienne un taux de soufre inférieur à 1,5 % en masse par rapport à la masse totale de la lignine. En effet, les inventeurs ont observé, de manière tout à fait inattendue, que lorsque la lignine contient un taux de soufre égal ou supérieur à 1,5 % en masse par rapport à la masse totale de la lignine, la dépolymérisation de la lignine n'a pas lieu ou elle est partielle. Lorsque la dépolymérisation est partielle, elle conduit à des molécules de masse molaire moyenne en poids supérieure à 2000 g/mol pour les molécules sous forme silylée. Le taux de soufre de la lignine mise en œuvre dans le procédé de l'invention est donc avantageusement, égal ou supérieur à zéro et reste inférieur à 1,5 % en masse, par rapport à la masse totale de la lignine, comme défini ci-dessous :

$$0 \leq \text{taux de soufre de la lignine} < 1{,}5\ \%\ \text{en masse,}$$

par rapport à la masse totale de la lignine.

Dans l'étape de dépolymérisation, la réaction entre la lignine et le composé silane de formule (III), a lieu en présence d'un catalyseur.

Par catalyseur, au sens de l'invention, on entend tout composé capable de modifier, notamment en augmentant, la vitesse de la réaction chimique à laquelle il participe, et qui est régénéré à la fin de la réaction. Cette définition englobe à la fois les catalyseurs, c'est-à-dire les composés qui exercent leur activité catalytique sans avoir besoin de subir une quelconque modification ou conversion, et les composés (appelés également pré-catalyseurs) qui sont introduits dans le milieu réactionnel et qui y sont convertis en un catalyseur.

Il est en particulier nécessaire que le catalyseur soit choisi en tenant compte notamment de son encombrement stérique, de son aptitude à activer le silane et de sa solubilité dans le milieu réactionnel.

Dans l'étape de dépolymérisation du procédé de l'invention, le catalyseur peut être un catalyseur organique choisi parmi :

- les carbocations de formule $(X^1)_3C^+$ avec $X^1$ représentant un atome d'hydrogène, un groupe alkyle, un groupe aryle, un groupe alkoxy, un groupe silyle, un groupe siloxy et un atome d'halogène, tels que définis ci-dessus, lesdits carbocations étant choisis parmi le cation trityle ($(C_6H_5)_3C^+$), le tropilium $(C_7H_7)^+$, le cation benzylique ($C_6H_5CH_2^+$), le cation allylique ($CH_3$-$CH^+$-$CH$=$CH_2$), le méthylium ($CH_3^+$), le cyclopropylium ($C_3H_5^+$), le carbocation cyclopropylique de formule $C3H_5$-$C^+R^1R^2$ avec $R^1$ et $R^2$ comme définis ci-dessus, ledit carbocation cyclopropylique étant choisi parmi le carbocation diméthyle cyclopropylique et le carbocation dicyclopropylique, le cation triazabicyclodécène (TBD), l' acylium $(R^1$-$C$=$O)^+$ avec $R^1$ comme défini ci-dessus et choisi parmi le méthyle, le propyle et le benzyle, le benzénium $(C_6H_5)^+$, le cation norbornyle $(C_7H_{11})^+$ ;
- les oxoniums choisis parmi $(CH_3)O^+BF_4^-$ (sel de Meerwein) et $(CH_3CH_{2h}O^+BF_4^-$ ;
- un ion silylium $(R^5)_3Si^+$ avec $R^5$ tel que défini précédemment, par exemple, choisi parmi $Et_3Si^+$ et $Me_3Si^+$ ;
- les cations disilyles, de préférence les cations disilyles ayant un hydrure pontant choisis parmi les formules indiquées ci-dessous

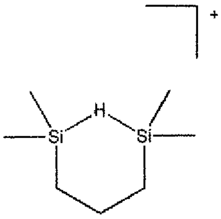
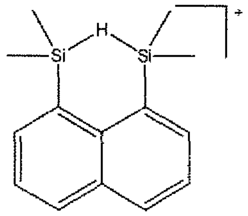
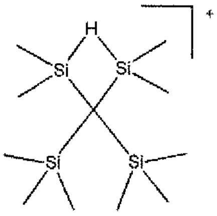

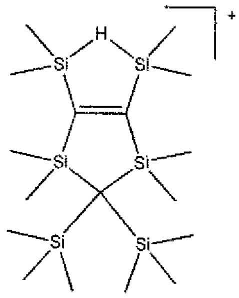

Les carbocations cités ci-dessus sont commerciaux ou peuvent être facilement synthétisés par l'homme du métier par différents procédés de synthèse, par exemple : le procédé de bassin de cation (cation pool), le procédé redox interne, le procédé utilisant un groupement partant, les procédés utilisant des acides de Lewis ou de Bronsted. Ces procédés sont décrits dans les références suivantes : R. R. Naredla et D. A. Klumpp, Chem. Rev. 2013, 113, pages 6905-6948 ; M. Saunders. et H. A. Jimenez-Vazquez, Chem. Rev. 1991, 91, pages 375-397.

Il est à noter que le contre ion anionique de l'ion silylium, des carbocations et des cations disilyles précités est, de préférence, un halogénure choisi parmi $F^-$, $Cl^-$, $Br^-$ et $I^-$, ou un anion choisi parmi $BF_4^-$, $SbF_6^-$, $B(C_6F_5)4^-$ , $B(C_6H_5)_4^-$ , $TfO^-$ou $CF_3SO_3^-$, $PF_6^-$.

Dans le procédé de l'invention, le catalyseur peut être également organométallique. A ce titre on peut citer les complexes d'iridium de formule (IV)

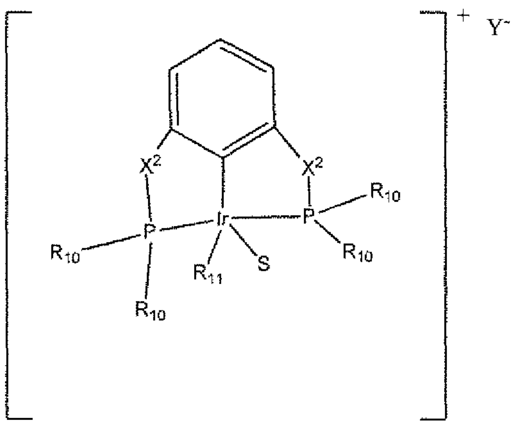

(IV)

dans laquelle

- $R^{10}$ représente un groupe alkyle ou aryle tel que défini précédemment, et de préférence un groupe tert-butyle ;
- $R^{11}$ représente un atome d'hydrogène ou un groupe alkyle tel que défini précédemment, et de préférence un atome d'hydrogène ;
- $X^2$ représente un groupe $-CH_2-$ ou un atome d'oxygène, et de préférence un atome d'oxygène ;
- $Y^-$ représente un contre ion choisi parmi $B(C_6F_5)_4^-$ et $B(C_6H_5)_4^-$, et de préférence $B(C_6F_5)_4^-$ ; et
- S représente une molécule de solvant, coordonnée au complexe, choisi parmi le diméthylesulfoxyde (DMSO), l'acétonitrile ($CH_3CN$) et l'acétone ($CH_3COCH_3$), et de préférence l'acétone.

De préférence, le catalyseur d'iridium est $[(POCOP)Ir(H)(acétonc)]^+B(C_6F_5)_4^-$ avec (POCOP) représentant le 2,6-bis(di-tert-butylphosphinito)phényle. Ce catalyseur peut être préparé selon les procédés décrits par I. Gottker-Schnetmann, P. White, et M. Brookhart, J. Am. Chem. Soc. 2004, 126, pages 1804-1811 ; et par J. Yang et M. Brookhart, J. Am. Chem. Soc. 2007, 129, pages 12656-12657.

Dans le procédé de l'invention, le catalyseur peut être également organométallique. A ce titre on peut citer les complexes de ruthénium de formule (V)

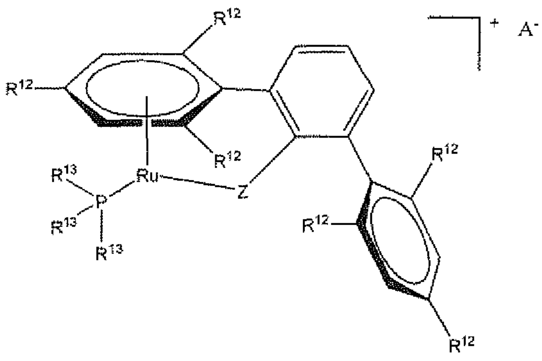

(V)

dans laquelle

- $R^{12}$ représente un atome d'hydrogène ou un groupe alkyle tel que défini précédemment, $R^{12}$ étant de préférence un groupe méthyle ;
- $R^{13}$ représente un aryle ou un groupe alkyle tel que défini précédemment, lesdits groupes aryle et alkyle étant éventuellement substitués, $R^{13}$ étant de préférence $p-FC_6H_4$ ;
- Z représente un groupe $-CH_2-$, un atome d'oxygène ou un atome de soufre, Z étant de préférence un atome de soufre ; et
- $A^-$ représente un contre ion choisi parmi $B(C_6F_5)_4^-$ et $[CHB_{11}H_5CL_6]^-$, $A^-$ étant de préférence $B(C_6F_5)_4^-$.

Ce type de catalyseur peut être préparé selon les procédés décrits par T. Stahl , H. F. T. Klare, and M. Oestreich, J. Am. Chem. Soc., 2013, 135, pages 1248-1251.

Le catalyseur peut également être de type acide de Lewis choisi parmi les catalyseurs organométalliques et métalliques. Les catalyseurs organométalliques et métalliques étant choisis parmi :

- les composés de bore de formule $B(X^3)_3$ avec $X^3$ représentant un atome d'hydrogène, un groupe alkyle, un groupe aryle, un groupe alkoxy tels que définis précédemment, lesdits composés de bore étant choisis parmi $BF_3$, $BF_3(Et_2O)$, $BCl_3$, $BBr_3$, le triphényle hydroborane, le tricyclohexyle hydroborane, $B(C_6F_5)_3$, le B-méthoxy-9-borabicyclo[3.3.1]nonane (B-méthoxy-9-BBN), le B-benzyl-9-borabicyclo[3.3.1]nonane (B-benzyl-9-BBN) ;
- les composés boréniums $R^1R^2B^+$ avec $R^1$ et $R^2$ tels que définis précédemment par exemple, $R^1$=$R^2$= phényle et $R^1$=$R^2$= méthylène (Y. Shoji, N. Tanaka, K. Mikami, M. Uchiyama and T. Fukushima, Nat. Chem., 2014, 6, 498-503), lesdits composés boréniums étant par exemple Me-TBD-BBN$^+$, les dérivés borenium ferrocène répondant à la formule

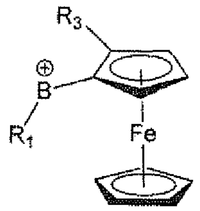

borenium ferrocène

dans laquelle $R^1$ et $R^3$ tels que définis précédemment, par exemple, $R^1$= phényle et $R^3$= 3,5-dimethylpyridyle (J. Chen, R. A. Lalancettea et F. Jäkle, Chem. Commun., 2013,49, pages 4893-4895) ;

- les composés de l'aluminium choisis parmi $AlCl_3$, $AlBr_3$, l'isopropoxyde d'aluminium Al(O-i-Pr)$_3$, l'éthanoate d'aluminium ($Al(C_2H_3O_2)$), le sel de Krossing $[Ag(CH_2Cl_2)]\{Al[OC(CF_3)_3]_4\}$, le $Li\{Al[OC(CF_3)_3]_4\}$, les composés cationiques d'aluminium de formule $(x^4)_2Al^+$ avec $X^4$ étant un atome d'halogène, un groupe alkoxy, un groupe alkyle tels que définis précédemment comme, par exemple, $Et_2Al^+$ ;
- les composés d'indium choisis parmi $InCl_3$, $In(OTf)_3$ ;
- les composés de fer choisis parmi $FeCl_3$, $Fe(OTf)_3$ ;
- les composés de l'étain choisis parmi $SnCL_4$, $Sn(OTf)_2$ ;
- les composés du phosphore choisis parmi $PCl_3$, $PCl_5$, $POCl_3$ ;
- les composés trifluorométhanesulfonates ou triflates ($CF_3SO_3^-$) de métaux de transitions et des lanthanides choisis parmi le triflate de scandium, le triflate de ytterbium, le triflate d'yttrium, le triflate de cérium, le triflate de samarium, le triflate de niodinium.

Dans le cadre de la présente invention OTf représente l'ion triflate ou trifluorométhanesulfonate de formule $CF_3SO_3^-$: les termes triflate ou trifluorométhanesulfonate, OTf ou $CF_3SO_3^-$peuvent donc être utilisés indifféremment pour désigner la même entité.

La préparation des dérivés borenium ferrocène est décrite par J. Chen, R. A. Lalancettea et F. Jäkle, Chem. Commun., 2013,49, pages 4893-4895 ; la préparation des sels de Krossing est décrite par I. Krossing, Chem.-Eur. J., 2001, 7, page 490 ; et la préparation de $Et_2Al^+$ est décrite par M. Khandelwal et R. J. Wehmschulte, Angew. Chem. Int. Ed. 2012, 51, pages 7323 -7326.

De préférence, le catalyseur est un catalyseur organométallique choisi parmi $BF_3$ ; $InCl_3$ ; le triphénylcarbénium tetrakis(perfluorophenyl)borate $[(Ph)_3C^+B(C_6F_5)_{,4}]^-$.

Certaines des abréviations utilisées dans le cadre de l'invention sont représentées dans la Figure 8 :

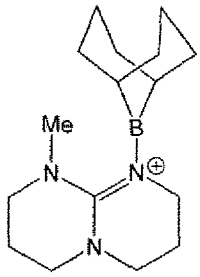

Me-TBD-BBN+

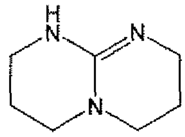

TBD

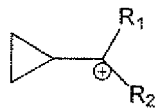

carbocation cyclopropyl

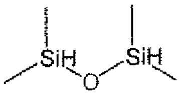

TMDS

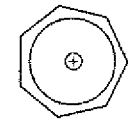

cation tropylium

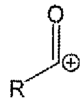

cation acylium

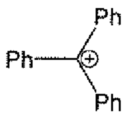

cation trityl

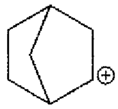

carbocations norbornyl

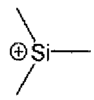

cation silylium

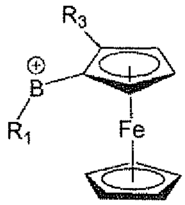

borenium ferrocène

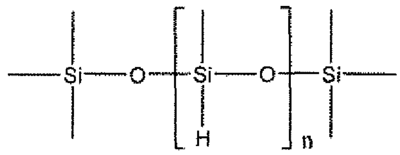

PMHS

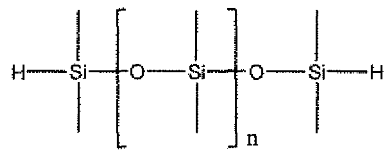

PDMS

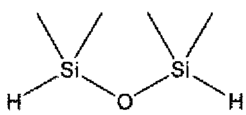

TMDS

Figure 8

Les catalyseurs peuvent, le cas échéant, être immobilisés sur des supports hétérogènes afin d'assurer une séparation facile dudit catalyseur et/ou son recyclage. Lesdits supports hétérogènes peuvent être choisis parmi les supports à base de gel de silice et de polymères plastiques comme, par exemple, le polystyrène ; les supports carbonés choisis parmi les nanotubes de carbone ; le carbure de silice ; l'alumine ; et le chlorure de magnésium ($MgCl_2$).

Dans l'étape de dépolymérisation du procédé selon l'invention, la réaction peut se produire sous une pression d'un ou d'un mélange de gaz inerte(s) choisi(s) parmi l'azote et l'argon, ou des gaz générés par le procédé notamment du méthane et d'hydrogène. La pression peut être comprise entre 0,2 et 50 bars, de préférence entre 0,2 et 30 bars, plus préférentiellement entre 1 et 20 bars, bornes incluses.

La température de la réaction peut être comprise entre 0 et 150°C, de préférence entre 0 et 125°C, plus préférentiellement entre 25 et 70°C, bornes incluses.

La durée de la réaction dépend du taux de conversion du composé silane de formule (III), de la nature de la

lignine ainsi que du taux de silylation souhaité.

La réaction peut être effectuée pendant une durée de 1 minute à 200 heures, avantageusement de 1 minute à 48 heures, de préférence de 10 minutes à 48 heures, bornes incluses.

Dans l'étape de dépolymérisation, en particulier la réaction entre les différents réactifs, peut avoir lieu dans un ou un mélange d'au moins deux solvant(s) choisi(s) parmi :

- les éthers silylés, de préférence, choisis parmi le 1,1,1,3,3,3-hexaméthyldisiloxane ($(Me_3Si)_2O$), le 1,1,1,3,3,3-hexaéthyldisiloxane ($(Et_3Si)_2O$).
- les hydrocarbures, de préférence, choisis parmi le benzène, le toluène, le pentane et l'hexane ;
- les sulfoxydes, de préférence, choisis parmi le diméthylesulfoxyde (DMSO) ;
- les halogénures d'alkyle, de préférence, choisis parmi le chloroforme, le chlorure de méthylène, le chlorobenzène, le dichlorobenzène.

Les silanes de formule (III) et les catalyseurs utilisés dans l'étape de dépolymérisation sont, en général, des composés commerciaux ou peuvent être préparés par les procédés connus de l'homme du métier.

Le rapport massique entre le composé silane de formule (III) et la lignine dépend du type de lignines employées et du type de molécules finales souhaitées (obtention des alcools primaires du type IIb, IId, IIf éventuellement silylés ou obtention des ethers silylés du type IIa, IIc, IIe, comme représentés dans les exemples). Les composés de formule (II) contenant une chaine propyle non substituée sont les produits de réduction des composés comprenant des groupements siloxy aliphatiques. La réduction de ces alcools silylés en alcanes se réalise par le même procédé que celui utilisé pour la dépolymérisation de la lignine. Comme le procédé de dépolymérisation réduit les liaisons carbone sp3-oxygène, les liaisons silylées (-C-O-Si-) peuvent être facilement réduites en alcane (-C-H) ou en alcool (-C-OH). La sélectivité alcool/alcane dépendra du nombre d'équivalents de composé silane de formule (III) ajouté.

Ainsi, dans le cadre de la présente invention, le rapport massique entre le composé silane de formule (III) et la lignine peut être compris entre 0,5 et 6, de préférence entre 1 et 4, bornes incluses.

La quantité de catalyseur utilisé dans l'étape de dépolymérisation est de 0,001 à 1 équivalent massique, de préférence de 0,001 à 0,9 équivalent en masse, plus préférentiellement de 0,01 à 0,9 équivalent massique, encore plus préférentiellement de 0,01 à 0,5 équivalent massique, bornes incluses, par rapport à la masse initiale de la lignine.

Après la dépolymérisation, les composés aromatiques résultants peuvent être sous forme silylée. Une simple hydrolyse dans des conditions bien connues de l'homme du métier peut alors conduire aux composés aromatiques correspondant sous leurs formes non silylées.

Dans le cadre de la présente invention, par hydrolyse on entend un procédé de transformation des groupements siloxy présents dans des composés aromatiques silylés issus de dépolymérisation de la lignine, en groupements hydroxyles, par une réaction de désilylation. Cette transformation peut se réaliser dans des conditions acides ou basiques ou bien en présence d'ions fluorures, ces conditions étant bien connues de l'homme du métier. Dans le cadre de la présente invention, le procédé d'hydrolyse est, de préférence, choisi parmi : HCl ou $H_2SO_4$ 2 M dans le THF ; NaOH ou KOH 10 % dans un mélange eau/THF ; le fluorure de tétra-*n*-butylammonium (TBAF) 1 M dans le THF.

Une simple filtration peut permettre de récupérer le catalyseur éventuellement supporté et d'éliminer les éventuels sous-produits.

Comme déjà indiqué, le procédé de l'invention permet la préparation des composés aromatiques monocycliques de formule (I) avec une bonne pureté, c'est-à-dire, une pureté supérieure ou égale à 90 % en masse, de préférence comprise entre 90 et 99,9% en masse, par rapport à la masse totale des composés aromatiques monocycliques de formule (I) ou pouvant être facilement purifiés. Dans le cas où une purification des composés aromatiques monocycliques de formule (I) s'avère nécessaire, des techniques de séparation bien connues de l'homme du métier et classiquement utilisées dans ce domaine, comme par exemple, la distillation ou la chromatographie sur colonne peuvent être mises en œuvre.

Dans le procédé de l'invention, le choix de l'espèce végétale dont sera extraite la lignine ainsi que son procédé d'extraction jouent un rôle important, notamment, sur la nature du composé aromatique de formule (I) que l'on cherche à obtenir et donc la sélectivité du procédé de l'invention, sur le rendement du procédé de l'invention, sur le degré de pureté dudit composé aromatique et sur la productivité de l'étape de dépolymérisation. Plus le pourcentage de résidu susceptible de donner la molécule désirée augmente dans l'espèce, plus la productivité du processus augmente.

La sélection de l'espèce végétale se réalise en tenant compte des paramètres décrits ci-après.

a) L'espèce végétale sélectionnée doit contenir avantageusement un pourcentage relativement élevé, c'est-à-dire au moins 10 % en masse de la lignine par rapport à la masse totale de l'échantillon de l'espèce végétale sélectionnée, ceci afin d'augmenter le rendement global de composé aromatique monocyclique de formule (I) final par rapport au matériau de départ, comme par exemple le bois.

b) L'espèce végétale doit être sélectionnée de sorte à avoir au moins 50% de résidu G, H ou S par rapport au

nombre total de résidus présents dans la lignine utilisée. Il est à noter que le pourcentage des résidus présents dans la lignine peut être déterminé par des techniques connues par l'homme du métier, par exemple, la pyrolyse, la RMN, etc. Ce paramètre joue un rôle important dans la sélectivité par rapport au composé aromatique monocyclique de formule (I) qui sera obtenu ainsi que dans l'augmentation du rendement dudit composé aromatique. Comme déjà indiqué, la lignine contient les résidus p-hydroxyphényle (H), guaiacyle (G), et syringyle (S). Toutefois, la complexité ainsi que la structure de la lignine dépend largement de son origine. Or, en se basant sur la classification des lignines, on peut identifier quatre types : G, GS, HGS et HG.
Le type G peut être distingué des autres étant donné que les espèces contenants ce type de lignine sont exclusivement faites de résidus de type G. Ce type de lignine est en général issu des gymnospermes (softwood ou bois mou) et plus précisément de la division des conifères (Pinophyta) qui compte 600-650 espèces végétale.
Pour les autres types de résidus, les espèces naturelles contiennent toujours des mélanges GS ou HG, et les lignines issues de ces espèces sont caractérisées par le rapport S/G ou H/G. Toutefois il existe des espèces qui peuvent être riche en résidu S ou H. A titre d'exemple, on peut citer l'eucalyptus commun (*Eucalyptus globulus*) (bois dur) qui a un rapport S/G de 6.
Il existe également des espèces végétales génétiquement modifiées de sorte à avoir préférentiellement un seul résidu très majoritaire. Par exemple Mansfield est passé d'un ratio S/G = 2 à un ratio S/G = 12 pour le peuplier (J. J. Stewart, T. Akiyama, C. Chapple, J. Ralph, and S. D. Mansfield, Plant Physiol. 2009, 150, pages 621-635). Il est à noter que même si ces rapports entre les résidus ne sont pas respectés, par exemple, dans le cas où un mélange de résidu est contenu dans l'espèce, la productivité diminuera mais les produits finaux pourront être séparés par une méthode connue par l'homme du métier par exemple la distillation ou la chromatographie sur colonne.
c) L'espèce doit de même être avantageusement sélectionnée de façon à maximiser, c'est-à-dire avoir au moins 30% de liaisons clivables par rapport au nombre total de liaisons présentes entre les motifs monomères dans la lignine. Au sens de l'invention, on entend par liaisons clivables, les liaisons carbone $sp^3$-oxygène qui pourront être clivées par l'étape de dépolymérisation et qui conduisent à la coupure de toutes les connections entre deux motifs monomères successifs présents dans la lignine. Dans le cadre de la présente invention, le clivage sélectif des liaisons carbone $sp^3$-oxygène vise les liaisons de types $\beta$-O-4 et $\alpha$-O-4. Dans le procédé de l'invention, la lignine mise en œuvre peut être, par exemple, une lignine contenant au moins 30% de liaison du type $\beta$-O-4 et/ou au moins 3% de liaisons $\alpha$-O-4. Dans le cas de la lignine de bois, les liaisons de types $\beta$-O-4 et $\alpha$-O-4 constituent entre 40 et 60 % des liaisons présentes. Les pourcentages indiqués correspondent au pourcentage d'un type de liaison par rapport au nombre total de liaisons présentes entre les motifs monomères dans la lignine. Ce pourcentage peut être déterminé par la RMN, la pyrolyse, par exemple.
La lignine comporte également des liaisons modifiables mais non clivables comme les liaisons $\beta$-5, $\beta$-1, $\beta$-$\beta$. L'étape de dépolymérisation de la lignine dans le procédé de l'invention modifie ces liaisons mais un lien est toujours conservé entre les monomères aromatiques de la lignine.
Enfin, la troisième catégorie de liaison regroupe les liaisons non clivables et non modifiables comme les liaisons 4-0-5, 5-5. Dans le cadre de la présente invention, ces liaisons sont inertes et restent intactes dans les conditions opératoires appliquées. Il est donc important de sélectionner une lignine avec le plus de liaisons clivables possible (au moins 30% de liaisons clivables par rapport au nombre total de liaisons présentes entre les motifs monomères dans la lignine) pour pouvoir réaliser une dépolymérisation réussie de la lignine en des fragments similaires aux monolignols de départ comme montrés dans la Figure 1.

De préférence, dans le procédé de l'invention, préalablement à l'étape de dépolymérisation de la lignine, l'espèce végétale dont provient la lignine est sélectionnée. L'espèce végétale est de préférence sélectionnée de façon à augmenter les liaisons clivables $\beta$-O-4 et $\alpha$-O-4 et de façon à avoir un type de résidu majoritaire H, G ou S dans la lignine.
Ainsi, l'espèce végétale est de préférence sélectionnée de façon à avoir :

- au moins 10 % en masse de la lignine par rapport à la masse totale de l'échantillon de l'espèce végétale sélectionnée ;
- au moins 30 % de liaisons clivables par rapport au nombre total de liaisons présentes entre les motifs de monomères dans la lignine ; et
- au moins 50 % de résidu G, H ou S du nombre total de résidus présents dans la lignine utilisée.

Les espèces végétales peuvent être choisies, par exemple, parmi

- les cèdres, les pins, les épicéas, les sapins pour viser la formation d'un composé aromatique monocyclique de formule (I) ayant une structure dérivée du motif G ; ou
- les peupliers, les chênes, les eucalyptus dans le but de générer un composé aromatique monocyclique de formule (I) ayant une structure dérivée du motif S.

Une fois l'espèce végétale sélectionnée, elle doit être traitée de manière à extraire la lignine. Au sens de l'invention, le procédé d'extraction de la lignine désigne les procédés organosolv qui correspondent aux procédés utilisant un ou plusieurs solvants organiques pour extraire la lignine (*i.e.* les procédés : Acetocell, Alcell, Acetosolv, ASAM, Organocell, Milox, Formacell, Batelle/Geneva phenol),

Les procédés organosolv sont décrits par les références suivantes :

a) Alcell : J, H. Lora, W. G. Glasser, J Polym Environ, 2002, 10, 39-48 ;
b) Acetocell : Bojan Jankovic, Bioresource Technol., 2011,102, 9763-9771 ;
c) Acetosolv : J. C. Parajo, J. L. Alonso, D. Vazquez, Bioresource Technology, 1993, 46, 233-240 ;
d) ASAM : I. Miranda, H. Pereira, Holzforschung, 2002, 56, 85-90 ;
e) Batelle/Genevaphenol : A. Johansson, O. Aaltonen, P. Ylinen, Biomass 1987, 13, 45-65 ;
f) Formacell : X.F. Sun, R.C. Sun, P. Fowler, M.S. Baird, Carbohydr. Polym., 2004, 55, 379-391 ;
g) Milox: P. Ligero, A. Vega, J.J. Villaverde, Bioresource Technol.,2010, 101, 3188-3193;
h) Organocell : A. Lindner, G. Wegener, J. Wood Chem. Technol. 1988, 8, 323 -340.

Dans le cas du bois, les procédés d'extraction précités permettent la séparation des trois principaux constituants du bois : la cellulose, l'hémicellulose et la lignine. Les procédés précités sont principalement basés sur des transformations chimiques ou thermochimiques, ce qui conduit à la modification de la structure de la lignine extraite. Cela signifie qu'un bois issu d'une même espèce peut engendrer différentes structures de lignine et ceci en fonction du procédé d'extraction employé.

Dans le cadre de la présente invention, le procédé d'extraction de la lignine est choisi de sorte à modifier le moins possible la structure de la lignine initiale présente dans l'espèce.

Ainsi la lignine issue du procédé d'extraction garde les mêmes types de fonctionnalités et les mêmes proportions des liaisons que ceux présents dans la lignine de départ. Cela contribue à l'augmentation du rendement global du procédé de l'invention ainsi que la sélectivité par rapport à un composé aromatique monocyclique de formule (I) donné. Le procédé d'extraction dans la présente est un procédé organosolv qui permettent d'obtenir des lignines dont la structure est très proche de celle de la lignine initiale présente dans l'espèce.

Dans le cadre de l'invention, le procédé d'extraction de la lignine peut également englober les procédés de traitement de la lignine dans le but d'introduire des fonctionnalisations chimiques, de changer les propriétés physiques et/ou de modifier la masse molaire moyenne de la lignine. La lignine est un polymère formé par une distribution de fragments polymériques ayant différentes masses molaires. La masse molaire moyenne de la lignine correspond donc à la moyenne des masses de ces fragments polymériques ; elle peut être calculée par rapport à la masse des fragments ou par rapport à leurs nombres. Ces procédés de traitements peuvent améliorer le rendement et la sélectivité du procédé de l'invention. Ainsi, à l'issue des procédés d'extraction précités, la lignine peut être traitée afin de modifier le ratio des résidus H, G et S qui la constituent. Cette modification peut aussi, dans certains cas, conduire à un enrichissement en un résidu donné et/ou en un type de liaison donné, par la suite, à augmenter la sélectivité ainsi que le rendement et réduire les étapes de purification conduisant au composé aromatique monocyclique final.

Comme déjà indiqué, il est essentiel que la lignine obtenue soit exempte de soufre, c'est-à-dire qu'elle contienne un taux de soufre inférieur à 1,5 % en masse, par rapport à la masse totale de la lignine. Le taux de soufre de la lignine utilisée dans le procédé de l'invention est donc avantageusement, égal ou supérieur à zéro et reste inférieur à 1,5 % en masse, par rapport à la masse totale de la lignine.

Le taux de soufre peut être déterminé par les techniques physiques et chimiques connues de l'homme du métier, comme par exemple l'analyse élémentaire, le dosage par chromatographie ionique, par spectrophotométrie infrarouge, par oxydation du soufre en $SO_2$ puis dosage de ce dernier par les techniques connues de l'homme du métier, comme par exemple, le dosage acidimétrique, le dosage iodométrique, le dosage complexométrique.

La lignine obtenue est alors dépolymérisée dans des conditions précisées précédemment.

Le procédé de l'invention permet donc d'obtenir des composés contenant un seul cycle aromatique de masse molaire moyenne en poids inférieure à 2000 g/mol, de préférence de masse molaire moyenne en poids inférieure à 1500g/mol sous forme silylée ou de masse molaire moyenne en poids inférieure à 400g/mol (soit un degré de polymérisation de 1 unité monomère), répondant à la formule (I). La nature du composé aromatique obtenu dépend de l'abondance des motifs G, H et S dans la lignine utilisée. Ces proportions G/H/S dépendent de l'espèce végétale ainsi que de la méthode d'extraction de la lignine. En conséquence, la nature des produits obtenus dépend de l'origine de la lignine ainsi que de sa méthode d'extraction. Dans le cas de l'utilisation d'un silane de formule (III) et d'un catalyseur, la dépolymérisation de la lignine permet l'obtention de produits purs, c'est-à-dire une pureté supérieure ou égale à 90 % en masse, de préférence comprise entre 90 et 99,9 % en masse, par rapport à la masse totale des composés aromatiques monocycliques de formule (I), avec de bons rendements (de 10 à 70 % en masse de composé aromatique monocyclique de formule (I) par rapport à la masse de lignine de départ, par exemple) et ceci dans des conditions réactionnelles douces.

Comme déjà indiqué, dans le procédé de l'invention, les produits secondaires éventuellement formés sont, en

général, des oligomères liés par des liaisons non clivables au sens de l'invention et ayant des propriétés physico-chimique très différentes de celles des composés aromatiques monocycliques désirés. Cela permet donc une séparation facile des composés aromatiques monocycliques de formule (I) des produits secondaires, par les techniques conventionnelles de purification (la chromatographie sur colonne ou la distillation, par exemple).

Ainsi, le procédé de l'invention permet à la lignine de devenir la principale source de composés aromatiques d'origine biologique pour l'industrie chimique. Des composés aromatiques de hautes valeurs ajoutées comme, par exemple, le benzène, le toluène, les xylènes (BTX), les coniférols substitués, le phénol, les polyols aromatiques, et les quinines peuvent ainsi être obtenus et utilisés dans la synthèse des résines phénol-formaldéhyde, des polymères polyoléfine-lignine, des polymères polyester-lignine, des polyuréthanes, des bio-plastiques, des résines époxydes.

Les composés aromatiques obtenus par le procédé de l'invention peuvent donc être utilisés comme matières premières dans les secteurs de la construction, dans l'industrie pétrochimique, électrique, électronique, de textile, aéronautique, pharmacutique, cosmétique, agrochimique.

L'invention a donc pour objet l'utilisation des composés aromatiques monocycliques de formule (I) obtenus par le procédé de l'invention dans la fabrication de combustibles, de composants électroniques, de polymères plastiques, de caoutchouc, de médicaments, de vitamines, de produits cosmétiques, de parfums, de produits alimentaires, de fils et fibres synthétiques, de cuirs synthétiques, de colles, de pesticides, d'engrais.

L'invention a pour objet l'utilisation du procédé de préparation de composés aromatiques monocycliques de formule (I) selon l'invention dans la fabrication de combustibles, de composants électroniques, de polymères plastiques, de caoutchouc, de médicaments, de vitamines, de produits cosmétiques, de parfums, de produits alimentaires, de fils et fibres synthétiques, de cuirs synthétiques, de colles, de pesticides, d'engrais.

L'invention a, également, pour objet un procédé de fabrication de combustibles, de composants électroniques, de polymères plastiques, de caoutchouc, de médicaments, de vitamines, de produits cosmétiques, de parfums, de produits alimentaires, de fils et fibres synthétiques, de cuirs synthétiques, de colles, de pesticides, d'engrais, caractérisé en ce qu'il comprend une étape de préparation de composés aromatiques monocycliques de formule (I) par le procédé selon l'invention.

D'autres avantages et caractéristiques de la présente invention apparaîtront à la lecture des exemples ci-dessous donnés à titre illustratif et non limitatif.

## EXEMPLES

La synthèse des molécules chimiques de formule générale (I), est effectuée en suivant une démarche intégrant trois étapes intégrant la sélection de l'espèce végétale, l'extraction de la lignine ainsi qu'une méthode de dépolymérisation réductrice convergente. Le choix de l'espèce végétale dont sera extraite la lignine, son procédé d'extraction ainsi que la dépolymérisation convergente jouent un rôle important, notamment, sur la nature du composé aromatique de formule (I) que l'on cherche à obtenir et donc la sélectivité du procédé de l'invention, sur le rendement du procédé de l'invention, sur le degré de pureté dudit composé aromatique et sur la productivité. L'étape de dépolymérisation de la lignine est effectuée en présence d'un catalyseur, en faisant réagir une lignine avec un taux de soufre inférieur à 1,5% en masse par rapport à la masse totale de la lignine, avec un composé silane de formule (III) selon le protocole expérimental suivant.

Les réactifs utilisés, notamment le composé silane de formule (III) et le catalyseur sont des produits commerciaux.

### Protocole expérimental général de synthèse des composés aromatiques monocycliques de formule (I) à partir de la lignine

1. Sous atmosphère inerte d'argon ou d'azote, le composé silane de formule (III), le catalyseur (de 1 à 0,001 équivalents molaires calculée par rapport au nombre de mole de lignine ajouté) et la moitié de la quantité de solvant sont mis sous agitation dans un récipient en verre de volume adapté. La concentration en silane dans le mélange réactionnel varie de 1,0-6,0 $mol.L^{-1}$ (concentration calculée sur la base de la moitié du volume final de solvant introduit).

2. D'autre part, dans un tube de Schlenk, la lignine organosolv (10 - 40 % d'équivalent en masse de silane ajouté), préalablement séchée pendant la nuit à l'aide d'une rampe à vide, est placée sous agitation avec la moitié restante de solvant.

3. La solution contenant le catalyseur et le composé silane de formule (III) est ajoutée lentement (temps d'ajout 15 minutes-1 heure) à l'aide d'une seringue et sous agitation, au tube de Schlenk. Ce dernier est laissé ouvert afin

d'évacuer les gaz produits par la réaction.

4. Après la fin de l'ajout de la solution et l'arrêt du dégagement gazeux, le tube de Schlenk est fermé et est laissé sous agitation. La lignine de départ est alors soluble presque totalement. Le suivi de réaction est réalisé par GC-MS.

5. Une fois la réaction terminée (temps de réaction de 1 à 72 heures), le solvant ainsi que les composés volatils sont évaporés à l'aide d'une rampe à vide ($10^{-2}$ mbar). Le liquide visqueux obtenu est purifié à l'aide d'une chromatographie sur gel de silice en utilisant un gradient d'élution de 100 : 0 jusqu'à 0 : 100 de pentane : $CH_2Cl_2$ pour les fractions apolaires, et un gradient d'élution de 100 : 0 à 0 : 100 de $CH_2Cl_2$ : AcOEt pour les fractions polaires. Dans le cas où une fraction est très polaire, l'élution peux se réaliser avec un mélange AcOEt : MeOH (50 : 50 à 0 : 100). Il est à noter qu'en fonction de l'application visée, l'étape de purification peut ou pas être omise.

6. Enfin, les différentes fractions issues de la colonne sont hydrolysées en milieu acide en utilisant HCl ou $H_2SO_4$ 2 M dans le THF, ou en milieu basique en utilisant NaOH ou KOH 15 à 30 % en masse, ou finalement à l'aide d'un réactif fluoré type : HF-pyridine, TBAF, CsF, $NH_4F$ pour fournir le produit hydrolysé correspondant.

Un ensemble de résultats est présenté ci-dessous, donnant des exemples de dépolymérisation de la lignine organosolv.

Les catalyseurs testés sont le $B(C_6F_5)_3$ ainsi que le complexe d'iridium (((POCOP)Ir(H)(acetone)$^+B(C_6F_5)_4^-$) dont la synthèse est décrite par I. Gottker-Schnetmann, P. White, et M. Brookhart, J. Am. Chem. Soc. 2004, 126, pages 1804-1811 ; et par J. Yang et M. Brookhart, J. Am. Chem. Soc. 2007,129, pages 12656-12657.

La lignine utilisée est issue de plusieurs procédés de type organosolv {a) Alcell : J. H. Lora, W. G. Glasser, J Polym Environ, 2002, 10, pages 39-48 ; b) Acetocell : Bojan Jankovic, Bioresource Technol., 2011,102, pages 9763-9771 ; c) Acetosolv : J. C. Parajo, J. L. Alonso, D. Vazquez, Bioresource Technology, 1993, 46, pages 233-240 ; d) ASAM : I. Miranda, H. Pereira, Holzforschung, 2002, 56, pages 85-90 ; e) Batelle/Genevaphenol : A. Johansson, O. Aaltonen, P. Ylinen, Biomass 1987, 13, pages 45-65 ; f) Formacell : X.F. Sun, R.C. Sun, P. Fowler, M.S. Baird, Carbohydr. Polym., 2004, 55, pages 379-391 ; g) Milox: P. Ligero, A. Vega, J.J. Villaverde, Bioresource Technol,2010, 101, pages 3188-3193 ; h) Organocell : A. Lindner, G. Wegener, J. Wood Chem. Technol. 1988, 8, pages 323 -340} et en particulier le procédé AVIDEL (décrit par H. Q. Lam, Y. Le Bigot, M. Delmas, G. Avignon, Industrial Crops and Products, 2001, 14, pages 139-144) qui constitue une version optimisée du procédé Formacell.

Les types de bois desquelles les lignines sont issues, sont sélectionnés en se basant sur l'approche décrite dans cette invention afin de maximiser le rendement ainsi que la sélectivité envers un produit.

**La synthèse de molécules aromatiques trisubstituées en utilisant l'approche convergente :**

En se basant sur les données présentes dans la littérature, les espèces pouvant donner des composés de formules IIa et IIb avec les meilleurs rendements et sélectivités sont celles contenant :

- une lignine de type G (selon le classement des lignines) ;
- au moins 10 % en masse de la lignine par rapport à la masse totale de l'échantillon de l'espèce végétale sélectionnée ;
- au moins 30 % de liaisons clivables par rapport au nombre total de liaisons présentes entre les motifs monomères dans la lignine.

Or, les espèces dérivant de la famille des Pinacées comme par exemple les pins, les sapins et les cèdres répondent à ces critères de sélection. De même un mélange commercial de plusieurs types de bois dérivant de cette famille a été employé (F315), pour montrer la versatilité ainsi que la robustesse de la méthode.

D'autre part, le procédé utilisé pour l'extraction de la lignine est choisi de sorte à modifier le moins possible la structure de la lignine de l'espèce sélectionnée. Ceci permet de garder les mêmes types de fonctionnalités et les mêmes proportions des liaisons présentes dans la lignine de départ, ce qui contribue à l'augmentation du rendement global ainsi que la sélectivité envers un produit. Pour ce faire les méthodes d'extraction de types organosolv ont été utilisées dans le cadre de cette invention, et en particulier la méthode AVIDEL, car elles sont connues pour donner des lignines dont la structure est très proche de la lignine initiale présente dans l'espèce.

Par ailleurs, la dépolymérisation de la lignine dans des conditions réductrices selon l'invention, permet de converger tous les fragments obtenus en un seul produit final. Ainsi, un silane de formule générale (III) avec un catalyseur sont utilisés, afin de réaliser la réaction de dépolymérisation ainsi que la réduction des fragments obtenus afin de converger vers un produit final. Le contrôle de la sélectivité envers les produits IIa et IIb se réalise par la variation de la quantité de silane ainsi que celle du catalyseur ajouté.

**EXEMPLE 1 : Synthèse du composé IIa à partir de la lignine issu du mélange de bois F315 (extraite par le procédé AVIDEL) et avec le triéthylsilane ($Et_3SiH$)**

La lignine issue de la sciure de bois F315 (bois mou) a été extraite par le procédé AVIDEL (optimisé dans la littérature pour l'extraction de la lignine issue de la paille), puis a été dépolymérisée en employant le mode opératoire générale décrit ci-dessus. Le triéthylsilane est employé comme réducteur avec un rapport massique entre triethylsilane et lignine compris entre 2,7 et 3,1. 20 % en masse de catalyseur (masse calculée par rapport à la masse de lignine ajoutée) sont nécessaire pour réaliser la transformation. L'ajout du mélange catalyseur - silane dure entre 30 et 45 minutes, la solution demeure de couleur marron foncée, et la réaction est laissée sous agitation pendant 24 h à 25°C. Après la fin de la réaction suivie de l'évaporation du solvant et des volatiles, un gel ayant une masse de 60 % de la masse de silane initialement ajoutée est obtenu. Ce produit est purifié en utilisant les mêmes conditions que celles décrites dans le mode opératoire général. A l'issu de cette purification, le composé IIa est obtenu avec une très grande pureté avec un rendement massique de 10 à 20% par rapport à la masse de lignine employée (non optimisé). Ce produit a été caractérisé par GC-MS, RMN $^{13}C$, RMN $^{1}H$ et par HR-MS. Enfin, l'hydrolyse des fractions issues de la purification se réalise en agitant à 25°C chaque fraction pendant 16 heures en présence d'une solution de 2 M de HCl dans le THF. Enfin, les polyols sont obtenus après l'évaporation du solvant et des composés volatils.

**EXEMPLE 2 : Synthèse du composé IIb à partir de la lignine issu du mélange de bois F315 (extraite par le procédé AVIDEL) et avec le triéthylsilane ($Et_3SiH$)**

Le même mode opératoire employé dans l'exemple 1, est utilisé. L'obtention du composé IIb, toutefois le rapport massique entre le triéthylsilane ($Ft_3SiH$) et la lignine introduite est de 2,5. Le rendement massique obtenu est compris entre 27 et 32 % par rapport à la masse de lignine de départ introduite, avec une très grande pureté (> 99,7 % en masse par rapport à la masse des composés de formule (II) obtenus).

**EXEMPLE 3 : Synthèse du composé IIa à partir de la lignine issu du pin (*Pinus pinea*) (extraite par le procédé AVIDEL) en utilisant le triéthylsilane ($Et_3SiH$)**

Le même mode opératoire employé pour la dépolymérisation du mélange de bois F315, est utilisé pour la dépolymérisation de la lignine issue du pin (bois mou). De même, les produits obtenus dans les deux cas (pin et F315) sont similaires et le composé IIa a été obtenu avec un rendement massique de 28 % par rapport à la masse de lignine de départ introduite, avec une très grande pureté (> 99,7 % en masse par rapport à la masse des composés de formule (II) obtenus).

**EXEMPLE 4 : Synthèse du composé IIa à partir de la lignine issu de l'Épicéa commun (*Picea Abies)* (extraite par le procédé AVIDEL) en utilisant le triéthylsilane ($Et_3SiH$)**

Le même mode opératoire employé pour la dépolymérisation du mélange de bois F315, est utilisé pour la dépolymérisation de la lignine issue du Epicéa commun (bois mou). De même, les produits obtenus dans les deux cas (Epicéa et F315) sont similaires et le produit **IIa** a été obtenu avec un rendement massique de 22 % par rapport à la masse de lignine de départ introduite.

**EXEMPLE 5: dépolymérisation de la lignine issue du mélange de sciure de bois F315[28] (extraite par le procédé AVIDEL) le en utilisant le TMDS**

Dans le cas où TMDS (tétramethyldisiloxane) est utilisé comme silane, il y a possibilité de formation de gel, ce qui rend la réaction très difficile. Pour ce faire deux solutions peuvent être envisagées : la dilution de la solution 3 à 4 fois en employant le $CH_2Cl_2$ comme solvant ou bien l'utilisation du benzène ou du toluène comme solvant. Toutefois la réaction sera plus lente dans les deux cas envisagés. Si la réaction se réalise dans $CH_2Cl_2$, la concentration en TMDS est de l'ordre 1 - 3 $mol.L^{-1}$ (concentration calculée sur la base de la moitié du volume final de solvant introduit), 20 % en masse de $B(C_6F_5)_3$ (masse calculée par rapport à la masse de lignine ajoutée) sont nécessaires pour catalyser la réaction. La masse de lignine ajoutée est entre 10 à 30 % de la masse du silane ajouté. Le temps d'ajout du mélange catalyseur - silane s'étale entre 30 et 45 minutes. Ensuite, la réaction est laissée sous agitation pendant 24 heures à 25°C. Après la fin de la réaction, les composés volatils ainsi que le solvant sont évaporés sous vide ($10^{-2}$ mbar). Le mélange issu de la dépolymérisation se dégrade lors de sa purification sur colonne de silice et le produit II est hydrolysé en milieu basique, en utilisant un mélange de THF et de $H_2O$ contenant 10 % en masse de NaOH. Après 16 heures d'agitation à 25°C, les composés volatils ainsi que les solvants sont évaporés, et le produit est purifié sur colonne de silice.

**EXEMPLE 6 : dépolymérisation de la lignine issu du mélange de sciure bois commercial F315 (extraite par le procédé AVIDEL) en utilisant ([(POCOP)Ir(H)(acétone)]$^+$B$(C_6F_5)_4$) et le diéthylsilane comme réducteur**

La dépolymérisation du mélange de bois F315 est réalisée en suivant le mode opératoire générale de dépolymérisation (décrit ci-dessus). Dans le cas où le complexe ([(POCOP)Ir(H)(acétone)]$^+$B$(C_6F_5)_4$-) est utilisé pour la dépolymérisation de lignine, le mode opératoire est similaire à celui ou le $B(C_6F_5)_3$ est employé. $Et_2SiH_2$ (5 mol.L$^{-1}$) est utilisé comme agent de réduction dans le chlorobenzène. La masse de lignine correspond à 30 % de la masse du silane ajouté. La réaction se réalise en présence de 25 % en masse de catalyseur (masse calculée par rapport à la masse de lignine ajoutée). Le temps d'ajout du silane et du catalyseur est de 30 minutes. Le temps de réaction est de l'ordre de 24 heures. Ensuite, le solvant et les volatiles sont évaporés, et le liquide visqueux obtenu est purifié sur colonne de silice (voir mode opératoire général). L'hydrolyse des produits issus de la colonne est réalisée en agitant les produits pendant 16h dans une solution de 2 M de HCl dans le THF. Enfin, les différents polyols correspondants sont obtenus par évaporation du solvant et des volatils sous vide.

**La synthèse de molécules aromatiques tétrasubstituées en utilisant l'approche convergente :**

Les espèces pouvant données des molécules de formule IIc et IId avec les meilleurs rendements et sélectivités sont celles contenant :

- une lignine de type GS (selon le classement des lignines), avec un rapport S/G supérieur à 1 ;
- au moins 10 % en masse de la lignine par rapport à la masse totale de l'échantillon de l'espèce végétale sélectionnée ;
- au moins 30 % de liaisons clivables par rapport au nombre total de liaisons présentes entre les motifs monomères dans la lignine.

Or, quelques espèces dérivant du sous embranchement des angiospermes, par exemple, le hêtre, le bouleau, l'eucalyptus, le chêne répondent à ces critères de sélection.

D'autre part, le procédé utilisé pour l'extraction de la lignine est le même que celui choisi pour la synthèse des composés IIa et IIb.

L'étape de dépolymérisation de la lignine est celle employée pour la synthèse des composés IIa et IIb. Le contrôle de la sélectivité envers les produits IIc et IId se réalise par la variation de la quantité de silane ainsi que celle du catalyseur ajouté. Les meilleurs résultats ont été obtenus avec le chêne vert (Quercus ilex).

**EXEMPLE 7 : Synthèse du composé IIc à partir de la lignine issu du Chêne vert (*Quercus ilex*) (extraite par le procédé AVIDEL) en utilisant le triéthylsilane ($Et_3SiH$)**

La dépolymérisation du Chêne vert (bois dur) est réalisée en suivant le mode opératoire général de dépolymérisation décrit ci-dessus. Le rapport massique entre triéthylsilane et la lignine est compris entre 2,7 et 3,1. Le solvant utilisé est le $CH_2Cl_2$. La réaction se réalise en présence de 30-40 % en masse de catalyseur (masse calculée par rapport à la masse de lignine ajoutée). La solution de silane et de catalyseur est ajoutée sur une période de 30 minutes dans le tube de Schlenk et la réaction est laissée sous agitation pendant 24 heures à 25°C. Après la fin de la réaction et l'évaporation du solvant et des volatiles, le liquide visqueux obtenu contient essentiellement les composés IIa et IIc avec les proportions relatives 82 : 18 IIc : IIa. Le mélange est purifié en utilisant les mêmes conditions que décrites précédemment. Le rendement massique obtenu en composé IIc est de 35 % par rapport à la masse de lignine de départ introduite. Enfin, les composés IIa et IIc purifiés ont été hydrolysés en milieu acide en utilisant une solution de 2 M de HCl dans le THF. Apres 16 heures d'agitation à température ambiante (20 $\pm$ 5°C), le solvant ainsi que les volatils sont évaporées, pour fournir les différents polyols correspondants.

**EXEMPLE 8 : Synthèse du composé IId à partir de la lignine issu du Chêne vert (*Quercus ilex)* (extraite par le procédé AVIDEL) en utilisant le triéthylsilane ($Et_3SiH$)**

Le même mode opératoire employé dans l'exemple 1, est utilisé l'obtention du composé IIb, toutefois le rapport massique entre le triéthylsilane ($Et_3SiH$) et la lignine introduite est de 2,5 à 2,7. Le rendement massique obtenu est compris entre 30 et 34 % par rapport à la masse de lignine de départ introduite, avec une très grande pureté (> 99,7 %).

**EXEMPLE 9 (comparatif) : Dépolymérisation avec le triéthylsilane ($Et_3SiH$) de la lignine commerciale (Aldrich : lignine Kraft) issue du bois tendre puis désulfurée à l'aide de la soude**

Le même mode opératoire employé pour la dépolymérisation de la lignine issue de la sciure de bois F315

(exemple 1) est utilisé pour la dépolymérisation de la lignine issue du procédé Kraft et ayant un taux de soufre de 3,76 % en masse par rapport à la masse totale de la lignine. Aucune solubilisation ou dépolymérisation n'a été observée dans le cas de cette lignine. Dans le cas où ce même échantillon de lignine est retraité par le procédé AVIDEL, vu que ce traitement permet la diminution du degré de polymérisation de la lignine ainsi que la modification de sa structure, ce qui permet une dépolymersation plus facile de la lignine. Il est à noter que le taux de soufre atteint 3 % en masse par rapport à la masse totale de la lignine. Malgré le pré-traitement AVIDEL, la dépolymérisation ne se réalise toujours pas. Ceci implique que la présence de soufre dans le milieu réactionnel joue un rôle crucial dans la désactivation de la réaction.

Les produits de de formules IIa à IIf sont identifiés et caractérisés par analyse élémentaire, par RMN $^1$H, RMN $^{13}$C et par HR-MS (Appareil Shimadzu GCMS-QP2010 Ultra gas chromatograph mass spectrometer équippé avec une colonne capillaire de silice fisionnée : fused silica capillary column Supelco SLB™-ms (30 m x 0.25 mm x 0.25 $\mu$m).

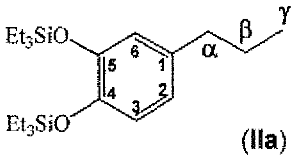
**(IIa)**

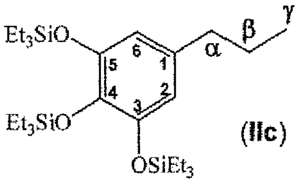
**(IIc)**

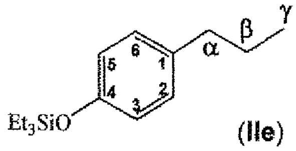
**(IIe)**

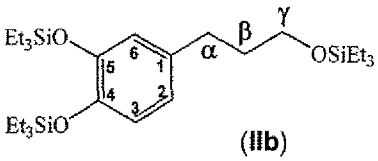
**(IIb)**

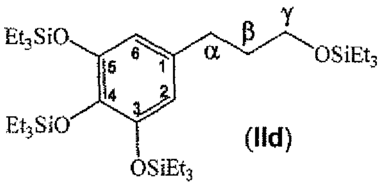
**(IId)**

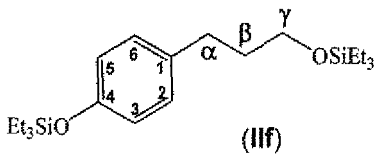
**(IIf)**

**IIa** :

**$^1$H NMR** (200 MHz, $CDCl_3$, $Me_4Si$) $\delta$ (ppm) = 6.71 (1 H, d, $^3J$ = 8.1 Hz, Ar-H), 6.63 (1 H, s, Ar-H), 6.58 (1 H, d, $^3J$ = 8.1 Hz, Ar-H), 2.45 (2 H, t, $^3J$ = 7.8 Hz, Ar-$CH_2$), 1.57 (2 H, sex, $^3J$= 7.8 Hz, $CH_2$-$CH_3$), 0.98 (18 H, t, $^3J$ = 7.9 Hz, $CH_3CH_2Si$), 0.90 (3 H, t, $^3J$ = 7.8 Hz, $CH_3CH_2Si$), 0.74 (12 H, q, $^3J$ = 7.9 Hz, $CH_3CH_2Si$).
**$^{13}$C NMR** (50 MHz, $CDCl_3$, $Me_4Si$): $\delta$ (ppm) =146.5, 144.7, 136.0, 121.3, 120.9, 120.2, 37.4, 24.7, 13.9, 6.9, 5.3, 5.2.
**HR-MS (APPI):** calculé (M +) ($C_{21}H_{40}O_2Si_2$), m/z 380.2566; trouvé (M +), m/z 380.2559. **Anal**. **Calculée**. for $C_{21}H_{40}O_2Si_2$ (mol. wt. 380.72): C, 66.25; H, 10.59. **Trouvé:** C, 66.18; H, 10.46.
**MS:** IE (m/z): 380 (9); 351 (4); 207 (8) ; 117 (4) ; 116 (11) ; 115 (100) ; 88 (7) ; 87 (74) ; 59 (45); 58 (4).

**IIb :**

**$^1$H NMR** (200 MHz, $CDCl_3$, $Me_4Si$) $\delta$ (ppm) = 6.79 - 6.50 (3 H, m, Ar-H), 3.60 (2 H, t, $^3J$ = 6.6 Hz, $CH_2$**O),** 2.54 (2 H, t, $^3J$ = 7.6 Hz, Ar-$CH_2$), 1.79 (2 H, quin, $^3J$ = 7.0 Hz, Ar-$CH_2$-$CH_2$), 1.05 - 0.88 (27 H, m, $CH_3CH_2Si$), 0.84 - 0.48 (18 H, m, $CH_3CH_2S_i$).
**$^{13}$C NMR** (50 MHz, $CDCl_3$, $Me_4Si$): $\delta$ (ppm) =146.5, 144.8, 135.4, 121.3, 120.9, 120.3, 62.3, 34.7, 31.5, 6.9, 6.8, 5.2, 5.2, 4.6.
MS: IE (m/z): 87 (100), 115 (57), 59 (38), 89 (28), 207 (24), 32 (16), 235 (11), 88 (10), 337 (9), 511 (8), 116 (6), 86 (6).

**IIc :**

**$^1$H NMR** (200 MHz, $CDCl_3$, $Me_4Si$) $\delta$ (ppm) = 6.27 (2 H, s, Ar-H), 2.39 (2 H, t, $^3J$ = 7.5 Hz, Ar-$CH_2$), 1.69 - 1.45 (2 H, m, $CH_2$-$CH_3$), 1.1 - 0.84 (27 H, m, $CH_3CH_2Si$), 0.90 - 0.81 (3 H, m, $CH_3CH_2Si$), 0.83 - 0.65 (18 H, m, $CH_3cH_2si$).
$^{13}$C NMR (50 MHz, $CDCl_3$, $Me_4Si$): $\delta$ (ppm) = 147.8, 146.5, 134.5, 113.6, 37.7, 24.6, 13.7, 7.0, 6.8, 5.4,5.2.
MS: IE (m/z): 510 (8); 339 (4); 338 (10); 337 (31) ; 116 (7) ; 115 (60); 88 (10) ; 87 (100); 86 (4) ; 59 (49).

**IId :**

**$^1$H NMR** (200 MHz, $CDCl_3$, $Me_4Si$) $\delta$ (ppm) = 6.28 (2 H, s, Ar-H), 3.59 (2 H, t, $^3J$ = 6.7 Hz, $CH_2$-O), 2.48 (2 H, t, $^3J$ = 7.5 Hz, Ar-$CH_2$), 1.78 (2 H, quin, $^3J$ = 7.3 Hz, Ar-$CH_2$-$CH_2$), 1.13 - 0.85 (36 H, m, $CH_3CH_2Si$), 0.84 - 0.49 (24 H, m, $CH_3CH_2Si$).
**$^{13}$C NMR** (50 MHz, $CDCl_3$, $Me_4Si$): $\delta$ (ppm) =147.9, 136.6, 134.0, 113.6, 62.3, 34.6, 31.7, 6.9, 6.8, 5.4, 5.2, 4.5.
**MS**: IE (m/z): 87 (100), 115 (36), 59 (32), 89 (19), 641 (9), 88 (9), 467 (8), 365 (7), 337 (6), 642 (5), 640 (5), 116 (4).

**IIe** :

**$^1$H NMR** (200 MHz, $CDCl_3$, $Me_4Si$) $\delta$ (ppm) = 7.03 (2 H, d, $^3J$= 7.7 Hz, Ar-H), 6.77 (2 H, $^3J$= 7.7 Hz, Ar-H), 2.52 (2 H, t, $^3J$= 7.3 Hz, Ar-$CH_2$), 1.61 (2 H, sex, $^3J$ = 7.4 Hz, $CH_2$-$CH_3$), 1.09 - 0.86 (9 H, m, $CH_3CH_2Si$), 0.97 - 0.85 (3 H, m, $CH_3CH_2Si$), 0.83 - 0.64 (6 H, m, $CH_3CH_2Si$).
**$^{13}$C NMR** (50 MHz, $CDCl_3$, $Me_4Si$): $\delta$ (ppm) = 153.5, 135.5, 129.4, 119.7, 37.4, 24.9, 13.9, 6.8,5.1.
**MS**: IE (m/z): 250 (37), 222 (21), 221 (100), 193 (51), 165 (26), 163 (13), 151 (16), 135 (13), 91 (32), 87 (21), 82 (17), 59 (29), 43 (23).

**IIf :**

**$^1$H NMR** (200 MHz, $CDCl_3$, $Me_4Si$) $\delta$ (ppm) = 7.03 (2H, d, $^3J$= 8.7, Ar-H), 6.76 (2H, d, $^3J$ = 8.5, Ar-H), 3.61 (2H, t, $^3J$ = 6.8, $CH_2$-O), 2.60 (2H, t, J = 7.8, Ar-$CH_2$); 1.87 (2H, quin, $^3J$=7.5, Ar-$CH_2$-$CH_2$); 1.08 - 0.89 (18 H, m, $CH_3CH_2Si$), 0.81 - 0.50 (12 H, m, $CH_3CH_2Si$).
**$^{13}$C NMR** (50 MHz, $CDCl_3$, $Me_4Si$): $\delta$ (ppm) = 153.6, 134.9, 129.4, 119.8, 62.3, 34.7; 31.4, 7.0, 6.8, 5.1, 4.6.
MS: IE (m/z): 380 (6), 352 (11), 351 (26), 248 (53), 219 (57), 161 (18), 147 (21), 133 (21), 119 (10), 91 (18), 89 (100), 87 (55), 75 (16), 59 (45).

**Protocole expérimental d'hydrolyse de composés aromatiques silylés issus de la dépolymérisation réductrice de la lignine**

A une solution de **IIa** (380,7 mg; 1,0 mmol, 1 équivalent) dans 4 mL de THF, *n*$B1_4NF.3H_2O$ (315,5 mg, 2,1 mmol, 2,1 équiv) a été ajouté lentement (environ 5 min) sous argon. La solution a été agitée pendant 1 h à 20 °C. Ensuite, les volatils ont été évaporés sous vide et 4 mL de dichlorométhane ont été ajoutés. Enfin, le composé **IIa** a été purifié sur colonne de silice en utilisant un gradient d'élution allant de 50% dichlorométhane à 50% acétate d'éthyle. L'évaporation des solvants conduit à l'obtention de 4-propylbenzene-1,2-diol (141,5 mg; 0,9 mmol; 84%) sous forme d'une huile incolore.
Le Tableau 1 récapitule les résultats de de l'hydrolyse des molécules aromatiques silylées **IIa-IIf** issues de la dépolymérisation réductrice de la lignine des lignines des exemples indiqués ci-dessus.

Tableau 1 :

| **Molécule aromatique silylée** | **Quantité de TBAF (équiv.)** | **Apparence** | **Rendement isolé (%)** |
|---|---|---|---|
| **IIa** | 2,1 | Huile incolore | 84 |
| **IIb** | 3,1 | Huile incolore | 86 |
| **IIc** | 3,1 | Poudre blanche ou cristaux incolores | 94 |
| **IId** | 4,1 | Gomme blanche | 82 |
| **IIe** | 1,1 | Huile incolore | 77 |
| **IIf** | 2,1 | Poudre blanche | 92 |
| Après l'hydrolyse, toutes les liaisons O-Si se transforment en O-H. | | | |

## Revendications

1. Procédé de préparation de composés aromatiques monocycliques de formule (I)

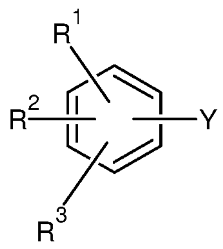

(I)

dans laquelle

- $R^1$, $R^2$, $R^3$, représentent, indépendamment l'un de l'autre, un atome d'hydrogène, un groupe hydroxyle, un groupe alkoxy, un groupe siloxy ;
- Y représente un groupe alkyle, un groupe alcényle, un groupe alcynyle, un groupe carbonyle $-CR^4=O$ avec $R^4$ représentant un atome d'hydrogène, un groupe alkyle, un groupe hydroxyle, un groupe alkoxy,

lesdits groupes alkyle, alcényle et alcynyle étant éventuellement substitués ;
**caractérisé en ce qu'**il comprend une étape de dépolymérisation de la lignine par clivage sélectif de la liaison carbone $sp^3$-oxygène des alkylaryles éthers du type β-O-4, α-O-4, β-5, β-1, β-β de la lignine présents dans la lignine, ladite étape de dépolymérisation comprenant la réaction,

- d'une lignine contenant un taux de soufre égal ou supérieur à zéro et inférieur à 1,5 % en masse en masse, par rapport à la masse totale de la lignine comme défini ci-dessous :

$$0 \leq \text{taux de soufre de la lignine} < 1{,}5\ \%\ \text{en masse},$$

par rapport à la masse totale de la lignine, avec
- un composé silane de formule (II)

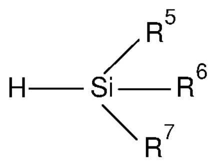

(III)

dans laquelle

- $R^5$, $R^6$ et $R^7$ représentent, indépendamment l'un de l'autre, un atome d'hydrogène, un atome d'halogène, un groupe hydroxyle, un groupe alkyle, un groupe alcényle, un groupe alcynyle, un groupe alkoxy, un groupe aryloxy, un groupe silylé, un groupe siloxy, un groupe aryle, un groupe amino, lesdits groupes alkyle, alcényle, alcynyle, alkoxy, silylé, siloxy, aryle et amino étant éventuellement substitués, ou
- $R^7$ est tel que défini ci-dessus et $R^5$ et $R^6$, pris ensemble avec l'atome de silicium auquel ils sont liés forment un hétérocycle silylé éventuellement substitué ;

en présence d'un catalyseur ; et **en ce que** les composés aromatiques monocycliques de formule (I) ont une masse molaire moyenne en poids inférieure à 1500g/mol sous forme silylée et une masse molaire moyenne en poids inférieure à inférieure 400 g/mol sous forme non silylée ; et
**en ce que** la lignine est extraite par les procédés organosolv.

2. Procédé selon la revendication 1, **caractérisé en ce que** dans le composé aromatique monocyclique de formule (I)

- $R^1$, $R^2$, $R^3$, représentent, indépendamment l'un de l'autre, un atome d'hydrogène ; un groupe hydroxyle ; un groupe alkoxy dont le groupe alkyle est choisi parmi les groupes méthyle, éthyle, propyle, butyle et ses isomères ramifiés ; un groupe siloxy ($-O-Si(X)_3$) dont chaque X, indépendamment l'un de l'autre, est choisi parmi un atome d'hydrogène, un groupe alkyle choisi parmi les groupes méthyle, éthyle, propyle, un groupe aryle choisi parmi le phényle et le benzyle, un organosilane polymérique de formule générale

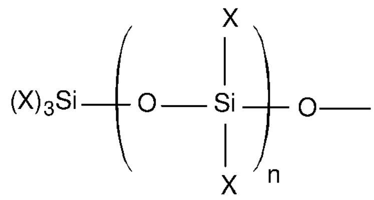

dans laquelle X est tel que défini ci-dessus et n est un nombre entier compris entre 1 et 20000, avantageusement entre 1 et 5000, plus avantageusement entre 1 et 1000 ;

- Y représente un groupe alkyle choisi parmi les groupes méthyle, éthyle, propyle, butyle et ses isomères ramifiés ; un groupe carbonyle $-CR^4=O$ avec $R^4$ représentant un atome d'hydrogène ; un groupe alkyle choisi parmi les groupes méthyle, éthyle, propyle, butyle et ses isomères ramifiés ; un groupe hydroxyle ;

lesdits groupes alkyle étant éventuellement substitués par un ou plusieurs groupes hydroxyle ; un ou plusieurs groupes siloxy ($-O-Si(X)_3$) dont chaque X, indépendamment l'un de l'autre, est choisi parmi un atome d'hydrogène, un groupe alkyle choisi parmi les groupes méthyle, éthyle, propyle, un groupe aryle choisi parmi le phényle et le benzyle, un organosilane polymérique de formule générale

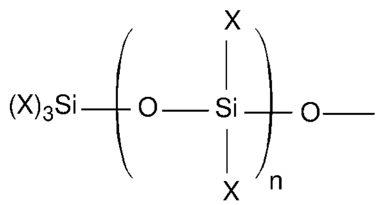

dans laquelle X est tel que défini ci-dessus et n est un nombre entier compris entre 1 et 20000, avantageusement entre 1 et 5000, plus avantageusement entre 1 et 1000.

**3.** Procédé selon l'une des revendications 1 ou 2, **caractérisé en ce que** dans le composé aromatique monocyclique de formule (I)

- $R^1$, $R^2$, $R^3$, représentent, indépendamment l'un de l'autre, un atome d'hydrogène ; un groupe hydroxyle ; un groupe alkoxy dont le groupe alkyle est un groupe méthyle ou un groupe éthyle ; un groupe siloxy ($-O-Si(X)_3$) dont chaque X, indépendamment l'un de l'autre, est un atome d'hydrogène, un groupe méthyle, un groupe éthyle, un groupe phényle, le polydiméthylsiloxane (PDMS), le polyméthylhydroxysiloxane (PMHS), le tétraméthyldisiloxane (TMDS) ;
- Y représente un groupe alkyle choisi parmi les groupes méthyle, éthyle, propyle, butyle et ses isomères ramifiés ; un groupe carbonyle $-CR^4=O$ avec $R^4$ représentant un atome d'hydrogène ; un groupe alkyle choisi parmi les groupes méthyle, éthyle, propyle, butyle et ses isomères ramifiés ; un groupe hydroxyle ;

lesdits groupes alkyle étant éventuellement substitués par un ou plusieurs groupes hydroxyle ; un ou plusieurs groupes siloxy ($-O-Si(X)_3$) dont chaque X, indépendamment l'un de l'autre, est choisi parmi un atome d'hydrogène, un groupe méthyle, un groupe éthyle, un groupe phényle, le polydiméthylsiloxane (PDMS), le polyméthylhydroxysiloxane (PMHS), le tétraméthyldisiloxane (TMDS).

**4.** Procédé selon l'une quelconque des revendications 1 à 3, **caractérisé en ce que** dans le composé silane de formule (III), $R^5$, $R^6$ et $R^7$ représentent, indépendamment l'un de l'autre, un atome d'hydrogène ; un groupe alkyle choisi parmi les groupes méthyle, éthyle, propyle, butyle et ses isomères ramifiés ; un groupe siloxy ($-O-Si(X)_3$) dont chaque X, indépendamment l'un de l'autre, est un groupe alkyle choisi parmi les groupes méthyle, éthyle, propyle, butyle et ses isomères ramifiés ; un groupe alkoxy dont le groupe alkyle est choisi parmi les groupes méthyle, éthyle,

propyle, butyle et ses isomères ramifiés ; un groupe aryle choisi parmi les groupes phényle et benzyle ; lesdits groupes alkyle et aryle étant éventuellement substitués.

5. Procédé selon l'une quelconque des revendications 1 à 4, **caractérisé en ce que** dans le composé silane de formule (III), $R^5$, $R^6$ et $R^7$ représentent, indépendamment l'un de l'autre, un atome d'hydrogène ; un groupe alkyle choisi parmi les groupes méthyle, éthyle, propyle ; un groupe aryle choisi parmi les groupes benzyle et phényle.

6. Procédé selon l'une quelconque des revendications 1 à 5, **caractérisé en ce que** la dépolymérisation de la lignine met en œuvre une lignine contenant au moins 30% de liaisons clivables par rapport au nombre total de liaisons présentes entre les motifs monomères dans la lignine.

7. Procédé selon l'une quelconque des revendications 1 à 6, **caractérisé en ce que** la dépolymérisation de la lignine met en œuvre une lignine contenant au moins 30% de liaison du type β-O-4 et/ou au moins 3% de liaisons α-O-4, les pourcentages indiqués correspondant au pourcentage d'un type de liaison par rapport au nombre total de liaisons présentes entre les motifs monomères dans la lignine.

8. Procédé selon l'une quelconque des revendications 1 à 7, **caractérisé en ce que** la dépolymérisation de la lignine met en œuvre un catalyseur organique choisi parmi :

- les carbocations choisis parmi le cation trityle ($(C_6H_5)_3C^+$), le tropilium $(C_7H_7)^+$, le cation benzylique ($C_6H_5CH_2^+$), le cation allylique ($CH_3$-$CH^+$-$CH$=$CH_2$), le méthylium ($CH_3^+$), le cyclopropylium ($C_3H_5^+$), le carbocation cyclopropylique choisi parmi le carbocation diméthyle cyclopropylique et le carbocation dicyclopropylique, le cation triazabicyclodécène (TBD), l'acylium $(R^1$-C=O$)^+$ avec $R^1$ choisi parmi le méthyle, le propyle et le benzyle, le benzénium $(C_6H_5)^+$, le cation norbornyle $(C_7H_{11})^+$ ;
- les oxoniums choisis parmi $(CH_3)_3O^+BF_4^-$ et $(CH_3CH_2)_3O^+BF_4^-$ ;
- un ion silylium choisi parmi $Et_3Si^+$ et $Me_3Si^+$ ;
- les cations disilyles ayant un hydrure pontant choisis parmi les formules indiquées ci-dessous

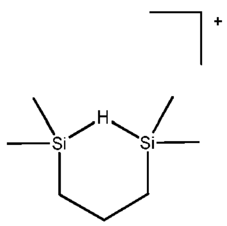
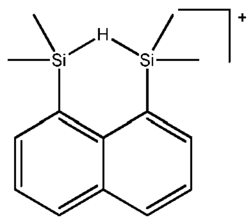
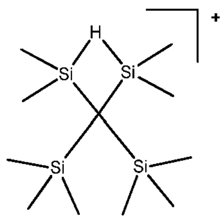

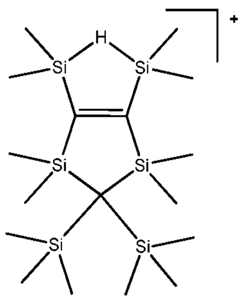

avec le contre ion dudit ion silylium, desdits carbocations et desdits cations disilyles étant

• un halogénure choisi parmi $F^-$, $Cl^-$, $Br^-$ et $I^-$; ou
• un anion choisi parmi BF4-, $SbF_6^-$, $B(C_6F_5)_4^-$, $B(C_6H_5)_4^-$, $CF_3SO_3^-$, $PF_6^-$.

9. Procédé selon l'une quelconque des revendications 1 à 7, **caractérisé en ce que** la dépolymérisation de la lignine met en œuvre un catalyseur organométallique choisi parmi :

- les complexes d'iridium de formule (IV),

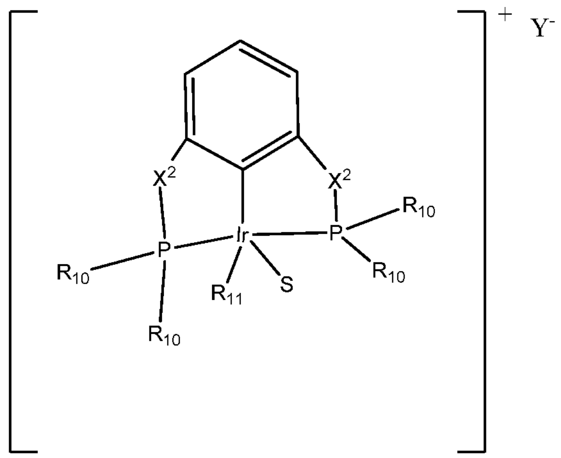

(IV)

dans laquelle

- $R^{10}$ représente un groupe alkyle ou aryle;
- $R^{11}$ représente un atome d'hydrogène ou un groupe alkyle ;
- $X^2$ représente un groupe $-CH_2-$ ou un atome d'oxygène ;
- $Y^-$ représente un contre ion choisi parmi $B(C_6F_5)4^-$ et $B(C_6H_5)_4^-$ ; et
- S représente une molécule de solvant, coordonnée au complexe, choisi parmi le diméthylesulfoxyde (DMSO), l'acétonitrile ($CH_3CN$) et l'acétone (CH3COCH3) ; et

- les complexes de ruthénium de formule (V)

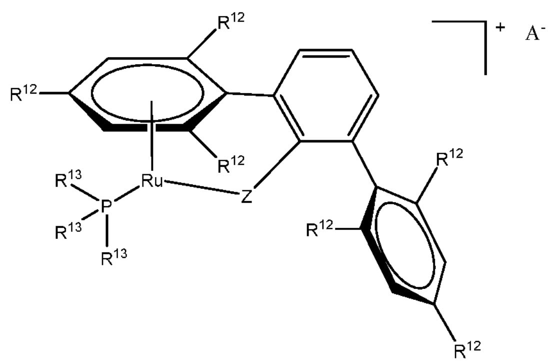

(V)

dans laquelle

- $R^{12}$ représente un atome d'hydrogène ou un groupe alkyle ;
- $R^{13}$ représente un aryle ou un groupe alkyle, lesdits groupes aryle et alkyle étant éventuellement substitués ;
- Z représente un groupe $-CH_2-$, un atome d'oxygène ou un atome de soufre ; et
- $A^-$ représente un contre ion choisi parmi $B(C_6F_5)_4^-$ et $[CHB_{11}H_5Cl_6]^-$.

**10.** Procédé selon la revendication 9, **caractérisé en ce que** le catalyseur organométallique est choisi parmi :

- le complexe d'iridium $[(POCOP)Ir(H)(acétone)]^+B(C_6F_5)_4^-$ avec (POCOP) représentant le 2,6-bis(di-tert-butylphosphinito)phényle ; et
- le complese de ruthénium de formule (V) dans laquelle

- $R^{12}$ représente un groupe méthyle ;
- $R^{13}$ représente $p$-$FC_6H_4$ ;
- Z représente un atome de soufre ; et
- $A^-$ représente $B(C_6F_5)_4^-$.

**11.** Procédé selon l'une quelconque des revendications 1 à 7, **caractérisé en ce que** la dépolymérisation de la lignine met en œuvre un catalyseur organométallique et métallique choisi parmi :

- les composés de bore choisis parmi $BF_3$, $BF_3(Et_2O)$, $BCl_3$, $BBr_3$, le triphényle hydroborane, le tricyclohexyle hydroborane, $B(C_6F_5)_3$, le B-méthoxy-9-borabicyclo[3.3.1]nonane (B-méthoxy-9-BBN), le B-benzyl-9-borabicyclo[3.3.1]nonane (B-benzyl-9-BBN) ;
- le composé borénium Me-TBD-BBN$^+$, les dérivés borenium ferrocène répondant à la formule

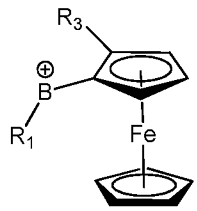

borenium ferrocène

- dans laquelle $R^1$= phényle et $R^3$= 3,5-dimethylpyridyle ;
- les composés de l'aluminium choisis parmi $AlCl_3$, $AlBr_3$, l'isopropoxyde d'aluminium $Al(O\text{-}i\text{-}Pr)_3$, l'éthanoate d'aluminium $(Al(C_2H_3O_2))$, le sel de Krossing $[Ag(CH_2Cl_2)]\{Al[OC(CF_3)_3]_4\}$, le $Li\{Al[OC(CF_3)_3]_4\}$, $Et_2Al^+$ ;
- les composés d'indium choisis parmi $InCl_3$, $In(OTf)_3$ ;
- les composés de fer choisis parmi $FeCl_3$, $Fe(OTf)_3$ ;
- les composés de l'étain choisis parmi $SnCl_4$, $Sn(OTf)_2$ ;
- les composés du phosphore choisis parmi $PCl_3$, $PCl_5$, $POCl_3$ ;
- les composés trifluorométhanesulfonates ou triflates ($CF_3SO_3^-$) de métaux de transitions et des lanthanides choisis parmi le triflate de scandium, le triflate de ytterbium, le triflate d'yttrium, le triflate de cérium, le triflate de samarium, le triflate de niodinium.

**12.** Procédé selon la revendication 11, **caractérisé en ce que** le catalyseur est choisi parmi $BF_3$ ; $InCl_3$ ; le triphénylcarbénium tetrakis(perfluorophenyl)borate $[(Ph)_3C^+B(C_6F_5)_4^-,B(C_6F_5)_3]$.

**13.** Procédé selon l'une quelconque des revendications 1 à 12, **caractérisé en ce que** dans l'étape de dépolymérisation la réaction est effectuée :

- sous une pression d'un ou d'un mélange de gaz inerte(s) choisi(s) parmi l'azote et l'argon, ou des gaz générés par le procédé notamment du méthane et d'hydrogène comprise entre 0,2 et 50 bars, bornes incluses ;
- à une température comprise entre 0 et 150°C, bornes incluses ;
- dans un ou un mélange d'au moins deux solvant(s) choisi(s) parmi :

  - les éthers silylés choisis parmi le 1,1,1,3,3,3-hexaméthyldisiloxane ($(Me_3Si)_2O$), le 1,1,1,3,3,3-hexaéthyldisiloxane ($(Et_3Si)_2O$) ;
  - les hydrocarbures choisis parmi le benzène, le toluène, le pentane et l'hexane ;
  - les sulfoxydes choisis parmi le diméthylesulfoxyde (DMSO) ;
  - les halogénures d'alkyle choisis parmi le chloroforme, le chlorure de méthylène, le chlorobenzène, le dichlorobenzène.

**14.** Procédé selon l'une quelconque des revendications 1 à 13 **caractérisé en ce que** dans l'étape de dépolymérisation le rapport massique entre le composé silane de formule (III) et la lignine est compris entre 0,5 et 6, bornes incluses.

**15.** Procédé selon l'une quelconque des revendications 1 à 14, **caractérisé en ce que** la quantité de catalyseur est de 0,001 à 1 équivalent massique, bornes incluses, par rapport à la masse initiale de la lignine.

**16.** Procédé selon l'une quelconque des revendications 1 à 15, **caractérisé en ce que** préalablement à l'étape de

dépolymérisation de la lignine, l'espèce végétale dont provient la lignine est sélectionnée de façon à avoir:

- au moins 10 % en masse de la lignine par rapport à la masse totale de l'échantillon de l'espèce végétale sélectionnée ;
- au moins 30 % de liaisons clivables par rapport au nombre total de liaisons présentes entre les motifs monomères dans la lignine ; et
- au moins 50 % de résidu G, H ou S du nombre total de résidus présents dans la lignine utilisée.

## Patentansprüche

**1.** Verfahren zur Herstellung von monocyclischen aromatischen Verbindungen der Formel (I)

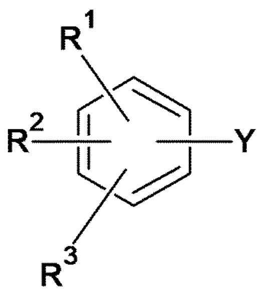

(I),

wobei

- $R^1$, $R^2$, $R^3$ unabhängig voneinander ein Wasserstoffatom, eine Hydroxylgruppe, eine Alkoxygruppe, eine Siloxygruppe darstellen;
- Y eine Alkylgruppe, eine Alkenylgruppe, eine Alkinylgruppe, eine Carbonylgruppe $-CR^4=O$ darstellt, wobei $R^4$ ein Wasserstoffatom, eine Alkylgruppe, eine Hydroxylgruppe, eine Alkoxygruppe darstellt,

die Alkyl-, Alkenyl- und Alkinylgruppen gegebenenfalls substituiert sind;
**dadurch gekennzeichnet, dass** es einen Schritt der Depolymerisation von Lignin durch selektive Spaltung der $sp^3$-Sauerstoff-Kohlenstoffbindung der Alkylarylether vom Typ β-O-4, α-O-4, β-5, β-1, β-β des Lignins umfasst, die im Lignin vorhanden sind, wobei der Depolymerisationsschritt die Reaktion

- eines Lignins mit einem Schwefelgehalt gleich oder größer null und weniger als 1,5 Massen-% bezogen auf die Gesamtmasse des Lignins, wie nachstehend definiert:
$0 \leq$ Schwefelgehalt von Lignin < 1,5 Massen-% bezogen auf die Gesamtmasse von Lignin, mit
- einer Silanverbindung der Formel (II) umfasst

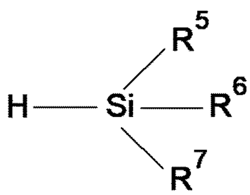

(III),

wobei

- $R^5$, $R^6$ und $R^7$ unabhängig voneinander ein Wasserstoffatom, ein Halogenatom, eine Hydroxylgruppe, eine Alkylgruppe, eine Alkenylgruppe, eine Alkinylgruppe, eine Alkoxygruppe, eine Aryloxygruppe, eine Silylgruppe, eine Siloxygruppe, eine Arylgruppe, eine Aminogruppe darstellen, wobei die Alkyl-, Alkenyl-, Alkinyl-, Alkoxy-, Silyl-, Siloxy-, Aryl- und Aminogruppen gegebenenfalls substituiert sind, oder
- $R^7$ wie oben definiert ist und $R^5$ und $R^6$ zusammen mit dem Siliciumatom, an das sie gebunden sind,

einen gegebenenfalls substituierten silylierten Heterocyclus bilden;

in Gegenwart eines Katalysators; und dass die monocyclischen aromatischen Verbindungen der Formel (I) eine gewichtsmittlere Molmasse von weniger als 1500 g/mol in silylierter Form und eine gewichtsmittlere Molmasse von weniger als 400 g/mol in nicht silylierter Form aufweisen; und
dass das Lignin durch Organosolv-Verfahren extrahiert wird.

**2.** Verfahren nach Anspruch 1, **dadurch gekennzeichnet, dass** in der monocyclischen aromatischen Verbindung der Formel (I)

- $R^1$, $R^2$, $R^3$ unabhängig voneinander ein Wasserstoffatom; eine Hydroxylgruppe; eine Alkoxygruppe, von der die Alkylgruppe ausgewählt ist aus Methyl-, Ethyl-, Propyl-, Butylgruppen und ihren verzweigten Isomeren; eine Siloxygruppe (-O-Si $(X)_3$), von der jedes X unabhängig voneinander ausgewählt ist aus einem Wasserstoffatom, einer Alkylgruppe, die ausgewählt ist aus Methyl-, Ethyl-, Propylgruppen, einer Arylgruppe, die ausgewählt ist aus Phenyl und Benzyl, einem polymeren Organosilan der allgemeinen Formel

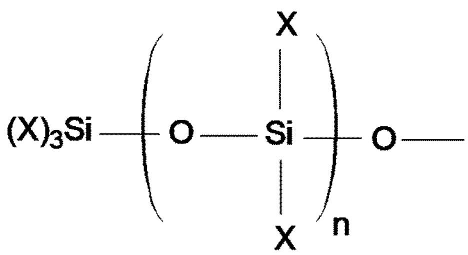

wobei X wie oben definiert ist, und n eine ganze Zahl zwischen 1 und 20000, vorteilhafterweise zwischen 1 und 5000, vorteilhafter zwischen 1 und 1000, ist;
- Y eine Alkylgruppe, die ausgewählt ist aus Methyl-, Ethyl-, Propyl-, Butylgruppen und ihren verzweigten Isomeren; eine Carbonylgruppe $-CR^4=O$, wobei $R^4$ ein Wasserstoffatom darstellt; eine Alkylgruppe, die ausgewählt ist aus Methyl-, Ethyl-, Propyl-, Butylgruppen und ihren verzweigten Isomeren; eine Hydroxylgruppe darstellt;

wobei die Alkylgruppen gegebenenfalls substituiert sind durch eine oder mehrere Hydroxylgruppen; eine oder mehrere Siloxygruppen ($-O-Si(X)_3$), von denen jedes X unabhängig voneinander ausgewählt ist aus einem Wasserstoffatom, einer Alkylgruppe, die ausgewählt ist aus Methyl-, Ethyl- und Propylgruppen, einer Arylgruppe, die ausgewählt ist aus Phenyl und Benzyl, einem polymeren Organosilan der allgemeinen Formel

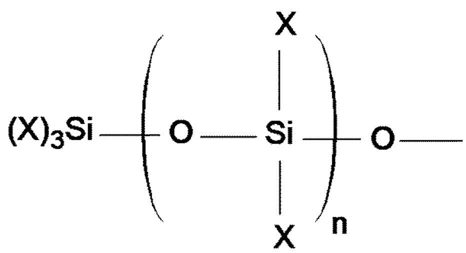

wobei X wie oben definiert ist, und n eine ganze Zahl zwischen 1 und 20000, vorteilhafterweise zwischen 1 und 5000, vorteilhafter zwischen 1 und 1000, ist.

**3.** Verfahren nach einem der Ansprüche 1 oder 2, **dadurch gekennzeichnet, dass** in der monocyclischen aromatischen Verbindung der Formel (I)

- $R^1$, $R^2$, $R^3$ unabhängig voneinander ein Wasserstoffatom; eine Hydroxylgruppe; eine Alkoxygruppe, von der die Alkylgruppe eine Methylgruppe oder eine Ethylgruppe ist; eine Siloxygruppe ($-O-Si(X)_3$), von der jedes X unabhängig voneinander ein Wasserstoffatom, eine Methylgruppe, eine Ethylgruppe, eine Phenylgruppe, Polydimethylsiloxan (PDMS), Polymethylhydroxysiloxan (PMHS), Tetramethyldisiloxan(TMDS) ist, darstellen;
- Y eine Alkylgruppe, die ausgewählt ist aus Methyl-, Ethyl-, Propyl-, Butylgruppen und ihren verzweigten Isomeren; eine Carbonylgruppe $-CR^4=O$, wobei $R^4$ ein Wasserstoffatom darstellt; eine Alkylgruppe, die ausgewählt aus Methyl-, Ethyl-, Propyl-, Butylgruppen und ihren verzweigten Isomeren; eine Hydroxylgruppe darstellt;

wobei die Alkylgruppen gegebenenfalls durch eine oder mehrere Hydroxylgruppen; eine oder mehrere Siloxygruppen (-O-Si$(X)_3$) substituiert sind von denen jedes X unabhängig voneinander ausgewählt ist aus einem Wasserstoffatom, einer Methylgruppe, einer Ethylgruppe, einer Phenylgruppe, Polydimethylsiloxan (PDMS), Polymethylhydroxysiloxan (PMHS), Tetramethyldisiloxan (TMDS).

**4.** Verfahren nach einem der Ansprüche 1 bis 3, **dadurch gekennzeichnet, dass** in der Silanverbindung der Formel (III) $R^5$, $R^6$ und $R^7$ unabhängig voneinander ein Wasserstoffatom; eine Alkylgruppe, die ausgewählt ist aus Methyl-, Ethyl-, Propyl-, Butylgruppen und ihren verzweigten Isomeren; eine Siloxygruppe (-O-Si $(X)_3$) darstellen, wobei jedes X unabhängig voneinander eine Alkylgruppe, die ausgewählt ist aus Methyl-, Ethyl-, Propyl-, Butylgruppen und ihren verzweigten Isomeren; eine Alkoxygruppe, von der die Alkylgruppe ausgewählt ist aus Methyl-, Ethyl-, Propyl-, Butylgruppen und ihren verzweigten Isomeren; eine Arylgruppe, die ausgewählt ist aus Phenyl- und Benzylgruppen, ist; wobei die Alkyl-und Arylgruppen gegebenenfalls substituiert sind.

**5.** Verfahren nach einem der Ansprüche 1 bis 4, **dadurch gekennzeichnet, dass** in der Silanverbindung der Formel (III) $R^5$, $R^6$ und $R^7$ unabhängig voneinander ein Wasserstoffatom; eine Alkylgruppe, die ausgewählt ist aus Methyl-, Ethyl- und Propylgruppen; eine Arylgruppe, die ausgewählt ist aus Benzyl- und Phenylgruppen, darstellen.

**6.** Verfahren nach einem der Ansprüche 1 bis 5, **dadurch gekennzeichnet, dass** bei der Depolymerisation des Lignins ein Lignin verwendet wird, das mindestens 30 % spaltbare Bindungen bezogen auf die Gesamtzahl der Bindungen enthält, die zwischen den Monomereinheiten in dem Lignin vorhanden sind.

**7.** Verfahren nach einem der Ansprüche 1 bis 6, **dadurch gekennzeichnet, dass** bei der Depolymerisation des Lignins ein Lignin verwendet wird, das mindestens 30% Bindung vom Typ β-O-4 und / oder mindestens 3% α-O-4-Bindungen enthält, wobei die angegebenen Prozentsätze dem Prozentsatz einer Art von Bindung im Verhältnis zur Gesamtzahl der Bindungen entsprechen, die zwischen den Monomereinheiten in Lignin vorhanden sind.

**8.** Verfahren nach einem der Ansprüche 1 bis 7, **dadurch gekennzeichnet, dass** die Depolymerisation des Lignins einen organischen Katalysator verwendet, der ausgewählt ist aus:

- den Carbokationen, die ausgewählt sind aus dem Tritylkation ($(C_6H_5)_3C^+$), Tropilium $(C_7H_7)^+$, dem Benzylkation ($C_6H_5CH_2^+$), dem Allylkation ($CH_3$-$CH^+$-$CH$=$CH_2$), Methylium ($CH_3^+$), Cyclopropylium ($C_3H_5^+$), Cyclopropylcarbokation, das ausgewählt ist aus Dimethylcyclopropylcarbokation und Dicyclopropylcarbokation, Triazabicyclodecenkation (TBD), Acylium $(R^{1-}C=O)^+$, wobei $R^1$ ausgewählt ist aus Methyl, Propyl und Benzyl, Benzol $(C_6H_5)^+$, dem Norbornylkation $(C_7H_{11})^+$;
- den Oxoniums, die ausgewählt sind aus $(CH_3)_3O^+BF_4^-$ und $(CH_3CH_2)_3O+BF_4^-$;
- einem Silyliumion, das ausgewählt ist aus $Et_3Si^+$ und $Me_3Si^+$;
- den Disilylkationen mit einem Brückenhydrid, die ausgewählt sind aus den unten angegebenen Formeln

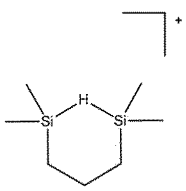
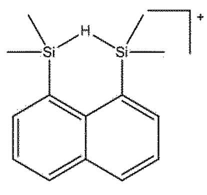
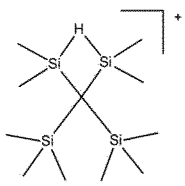

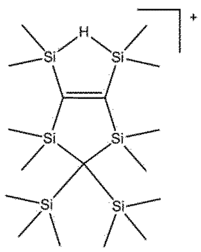

wobei das Gegenion des Silyliumions, der Carbokationen und der Disilylkationen ist

• in Halogenid, das ausgewählt ist aus $F^-$, $Cl^-$, $Br^-$ und $I^-$; oder
• ein Anion, das ausgewählt ist aus $BF_4^-$, $SbF_6^-$, $B(C_6F_5)_4^-$, $B(C_6H_5)_4^-$, $CF_3SO_3^-$, $PF_6^-$.

**9.** Verfahren nach einem der Ansprüche 1 bis 7, **dadurch gekennzeichnet, dass** bei der Depolymerisation des Lignins ein metallorganischer Katalysator verwendet wird, der ausgewählt ist aus:

- den Iridiumkomplexen der Formel (IV),

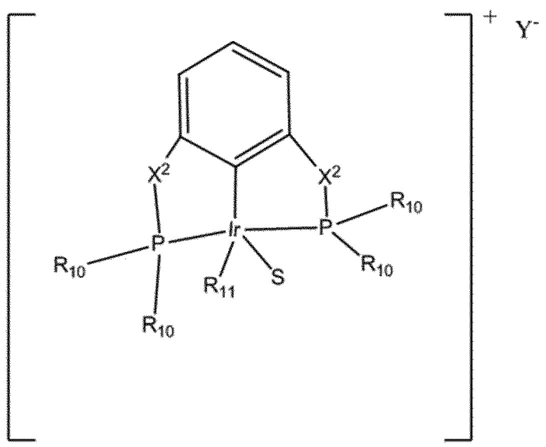

(IV),

wobei

- $R^{10}$ eine Alkyl- oder Arylgruppe darstellt;
- $R^{11}$ ein Wasserstoffatom oder eine Alkylgruppe darstellt;
- $X^2$ eine $-CH_2-$ Gruppe oder ein Sauerstoffatom darstellt;
- $Y^-$ ein Gegenion darstellt, das ausgewählt ist aus $B(C_6F_5)_4^-$ und $B(C_6H_5)_4^-$; und
- S ein an den Komplex koordiniertes Lösungsmittelmolekül darstellt, das ausgewählt ist aus Dimethylsulfoxid (DMSO), Acetonitril ($CH_3CN$) und Aceton ($CH_3COCH_3$); und
- dem Rutheniumkomplexen der Formel (V)

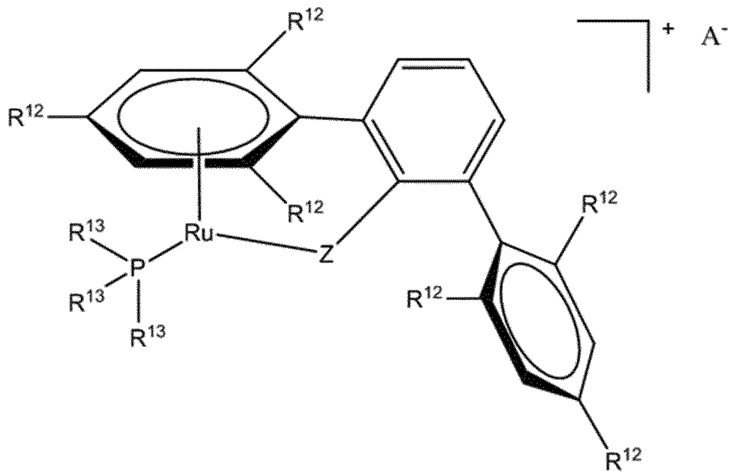

(V),

wobei

- $R^{12}$ ein Wasserstoffatom oder eine Alkylgruppe darstellt;

- $R^{13}$ eine Aryl- oder Alkylgruppe darstellt, wobei die Aryl- und Alkylgruppen gegebenenfalls substituiert sind;
- Z eine $-CH_2-$ Gruppe, ein Sauerstoffatom oder ein Schwefelatom darstellt; und
- $A^-$ ein Gegenion darstellt, das ausgewählt ist aus $B(C_6F_5)_4^-$ und $[CHB_{11} H_5Cl_6]^-$.

**10.** Verfahren nach Anspruch 9, **dadurch gekennzeichnet, dass** der metallorganische Katalysator ausgewählt ist aus:

- dem Iridiumkomplex $[(POCOP)Ir(H)(Aceton)]^+B(C_6F_5)4-$, wobei (POCOP) 2,6-Bis(di-tert-butylphosphinito)phenyl darstellt; und
- dem Rutheniumkomplex der Formel (V), wobei

  - $R^{12}$ eine Methylgruppe darstellt;
  - $R^{13}$ *p*-$FC_6H_4$ darstellt;
  - Z ein Schwefelatom darstellt; und
  - $A^-$ $B(C_6F_5)_4^-$ darstellt.

**11.** Verfahren nach einem der Ansprüche 1 bis 7, **dadurch gekennzeichnet, dass** bei der Depolymerisation des Lignins ein metallorganischer und metallischer Katalysator verwendet wird, der ausgewählt ist aus:

- den Borverbindungen, die ausgewählt sind aus $BF_3$, $BF_3(Et_2O)$, $BCl_3$, $BBr_3$, Triphenylhydroboran, Tricyclohexylhydroboran, $B(C_6F_5)_3$, B-Methoxy-9-borabicyclo[3.3.1]nonan (B-methoxy-9-BBN), B-Benzyl-9-borabicyclo[3.3.1]nonan (B-Benzyl-9-BBN);
- der Boreniumverbindung Me-TBD-BBN$^+$, wobei die Boreniumferrocenderivate der Formel entsprechen

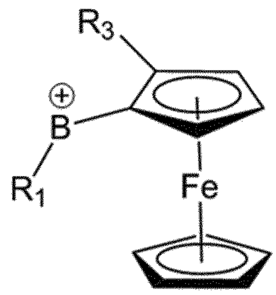

Boreniumferrocen

- wobei $R^1$ = Phenyl und $R^3$ = 3,5-Dimethylpyridyl;
- den Aluminiumverbindungen, die ausgewählt sind aus $AlCl_3$, $AlBr_3$, Aluminiumisopropoxid $A|(O\text{-}i\text{-}Pr)_3$, Aluminiumethanoat ($Al(C_2H_3O_2)$), Krossing-Salz $[Ag(CH_2Cl_2)]\{Al[OC(CF_3)_3]_4\}$, $Li\{Al[OC(CF_3)_3]_4\}$, $Et_2A^+$;
- den Indiumverbindungen, die ausgewählt sind aus $InCl_3$, $In(OTf)_3$;
- den Eisenverbindungen, die ausgewählt sind aus $FeCl_3$, $Fe(OTf)_3$;
- den Zinnverbindungen, die ausgewählt sind aus $SnCl_4$, $Sn(OTf)_2$;
- den Phosphorverbindungen, die ausgewählt sind aus $PCl_3$, $PCl_5$, $POCl_3$;
- den Trifluormethansulfonat- oder Triflat- ($CF_3SO_3^-$) Verbindungen von Übergangsmetallen und Lanthaniden, die ausgewählt sind aus Scandiumtriflat, Ytterbiumtriflat, Yttriumtriflat, Certriflat, Samariumtriflat, Niodiniumtriflat.

**12.** Verfahren nach Anspruch 11, **dadurch gekennzeichnet, dass** der Katalysator ausgewählt ist aus $BF_3$; $InCl_3$; Triphenylcarbeniumtetrakis(perfluorphenyl)borat $[(Ph)_3C^+B(C_6F_5)_4^-$, $B(C_6F_5)_3]$.

**13.** Verfahren nach einem der Ansprüche 1 bis 12, **dadurch gekennzeichnet, dass** im Depolymerisationsschritt die Reaktion durchgeführt wird:

- unter einem Druck zwischen 0,2 und 50 bar einschließlich der Grenzwerte in einem oder einer Mischung von Inertgasen, die aus Stickstoff und Argon ausgewählt sind, oder Gasen, die durch das Verfahren erzeugt werden, insbesondere Methan und Wasserstoff;
- bei einer Temperatur zwischen 0 und 150 °C einschließlich der Grenzwerte;
- in einem oder einer Mischung aus mindestens zwei Lösungsmitteln, das (die) ist (sind) ausgewählt aus:

- den Silylethern, die ausgewählt sind aus 1,1,1,3,3,3-Hexamethyldisiloxan ($(Me_3Si)_2O$), 1,1,1,3,3,3-Hexaethyldisiloxan ($(Et_3Si)_2O$);
- den Kohlenwasserstoffen, die ausgewählt sind aus Benzol, Toluol, Pentan und Hexan;
- Sulfoxiden, die ausgewählt sind aus Dimethylsulfoxid (DMSO);
- Alkylhalogenide, die ausgewählt sind aus Chloroform, Methylenchlorid, Chlorbenzol, Dichlorbenzol.

**14.** Verfahren nach einem der Ansprüche 1 bis 13, **dadurch gekennzeichnet, dass** im Depolymerisationsschritt das Massenverhältnis zwischen der Silanverbindung der Formel (III) und dem Lignin zwischen 0,5 und 6 einschließlich der Grenzwerte liegt.

**15.** Verfahren nach einem der Ansprüche 1 bis 14, **dadurch gekennzeichnet, dass** die Katalysatormenge 0,001 bis 1 Massenäquivalent einschließlich der Grenzwerte bezogen auf die Ausgangsmasse des Lignins beträgt.

**16.** Verfahren nach einem der Ansprüche 1 bis 15, **dadurch gekennzeichnet, dass** vor dem Depolymerisationsschritt des Lignins die Pflanzenart, aus der das Lignin stammt, ausgewählt ist, um Folgendes zu haben:

- mindestens 10 Massen-% Lignin bezogen auf die Gesamtmasse der Probe der ausgewählten Pflanzenart;
- mindestens 30 % spaltbare Bindungen im Verhältnis zur Gesamtzahl der Bindungen, die zwischen den Monomereinheiten im Lignin vorhanden sind; und
- mindestens 50 % der Rückstände G, H oder S der Gesamtzahl der Rückstände, die im verwendeten Lignin vorhanden sind.

## Claims

**1.** Method for preparing monocyclic aromatic compounds of formula (I)

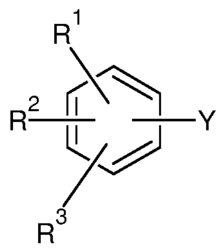

(I)

wherein

- $R^1$, $R^2$, $R^3$, represent, independently from one another, a hydrogen atom, a hydroxyl group, an alkoxy group, a siloxy group;
- Y represents an alkyl group, an alkenyl group, an alkynyl group, a carbonyl group $-CR^4=O$ with $R^4$ representing a hydrogen atom, an alkyl group, a hydroxyl group, an alkoxy group,

said alkyl, alkenyl and alkynyl groups being optionally substituted; **characterised in that** it comprises a step of depolymerising lignin by selective cleaving of the carbon $sp^3$-oxygen bond of the ether alkylaryls of the type β-O-4, α-O-4, β-5, β-1, β-β of the lignin present in lignin, said depolymerisation step comprising the reaction,

- of a lignin containing a sulphur ratio equal to or greater than zero and less than 1.5% by mass, with respect to the total mass of lignin as defined below:
$0 \leq$ sulphur ratio of lignin $< 1.5\%$ by mass,

with respect to the total mass of lignin, with

- a silane compound of formula (II)

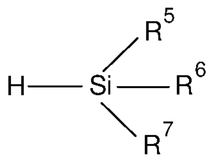

(III)

wherein

- $R^5$, $R^6$ and $R^7$ represent, independently from one another, a hydrogen atom, a halogen atom, a hydroxyl group, an alkyl group, an alkenyl group, an alkynyl group, an alkoxy group, an aryloxy group, a silyl group, a siloxy group, an aryl group, an amino group, said alkyl, alkenyl, alkynyl, alkoxy, silyl, siloxy, aryl and amino groups being possibly substituted, or
- $R^7$ is such as defined above and $R^5$ and $R^6$, taken together with the silicon atom to which they are bonded form a silyl heterocycle possibly substituted;

in the presence of a catalyst; and **in that** the monocyclic aromatic compounds of formula (I) have an average molar mass by weight less than 1500g/mol in silylated form and an average molar mass by weight less than 400g/mol in non-silylated form; and
**in that** the lignin is extracted by organosolv methods.

**2.** Method according to claim 1, **characterised in that** in the monocyclic aromatic compound of formula (I)

- $R^1$, $R^2$, $R^3$, represent, independently from one another, a hydrogen atom; a hydroxyl group; an alkoxy group of which the alkyl group is chosen from among the groups methyl, ethyl, propyl, butyl and the branched isomers thereof; a siloxy group ($-O-Si(X)_3$) of which each X, independently from one another, is chosen from among a hydrogen atom, an alkyl group chosen from among methyl, ethyl, propyl groups, an aryl group chosen from among phenyl and benzyl, a polymeric organosilane of general formula

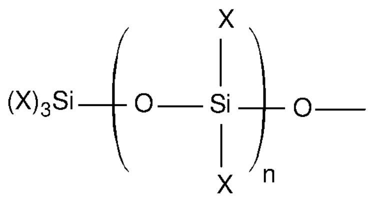

wherein X is such as defined above and n is an integer comprised between 1 and 20,000, advantageously between 1 and 5,000, more advantageously between 1 and 1,000;
- Y represents an alkyl group chosen from among the groups methyl, ethyl, propyl, butyl and the branched isomers thereof; a carbonyl group $-CR^4=O$ with $R^4$ representing a hydrogen atom; an alkyl group chosen from among the groups methyl, ethyl, propyl, butyl and the branched isomers thereof; a hydroxyl group;
said alkyl groups being optionally substituted by one or more hydroxyl groups; one or more siloxy groups ($-O-Si(X)_3$) of which each X, independently from one another, is chosen from among a hydrogen atom, an alkyl group chosen from among methyl, ethyl, propyl groups, an aryl group chosen from among phenyl and benzyl, a polymeric organosilane of general formula

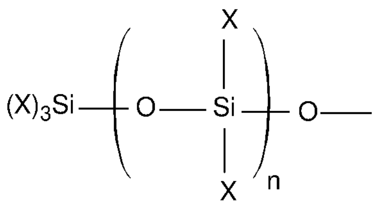

wherein X is such as defined above and n is an integer comprised between 1 and 20,000, advantageously between 1 and 5,000, more advantageously between 1 and 1,000.

**3.** Method according to one of claims 1 or 2, **characterised in that** in the monocyclic aromatic compound of formula (I)

- $R^1$, $R^2$, $R^3$, represent, independently from one another, a hydrogen atom; a hydroxyl group; an alkoxy group of which the alkyl group is a methyl group or an ethyl group; a siloxy group (-O-Si(X)$_3$) of which each X, independently from one another, is a hydrogen atom, a methyl group, an ethyl group, a phenyl group, polydimethylsiloxane (PDMS), polymethylhydroxysiloxane (PMHS), tetramethyldisiloxane (TMDS);
- Y represents an alkyl group chosen from among the groups methyl, ethyl, propyl, butyl and the branched isomers thereof; a carbonyl group - $CR^4$=O with $R^4$ representing a hydrogen atom; an alkyl group chosen from among the groups methyl, ethyl, propyl, butyl and the branched isomers thereof; a hydroxyl group;

said alkyl groups being possibly substituted by one or more hydroxyl groups; one or more siloxy groups (-O-Si(X)$_3$) of which each X, independently from one another, is chosen from among a hydrogen atom, a methyl group, an ethyl group, a phenyl group, polydimethylsiloxane (PDMS), polymethylhydroxysiloxane (PMHS), tetramethyldisiloxane (TMDS).

**4.** Method according to any one of claims 1 to 3, **characterised in that** in the silane compound of formula (III), $R^5$, $R^6$ and $R^7$ represent, independently from one another, a hydrogen atom; an alkyl group chosen from among the groups methyl, ethyl, propyl, butyl and the branched isomers thereof; a siloxy group (-O-Si(X)$_3$) of which each X, independently from one another, is an alkyl group chosen from among the groups methyl, ethyl, propyl, butyl and the branched isomers thereof; an alkoxy group of which the alkyl group is chosen from among the groups methyl, ethyl, propyl, butyl and the branched isomers thereof; an aryl group chosen from among phenyl and benzyl groups; said alkyl and aryl groups being optionally substituted.

**5.** Method according to any one of claims 1 to 4, **characterised in that** in the silane compound of formula (III), $R^5$, $R^6$ and $R^7$ represent, independently from one another, a hydrogen atom; an alkyl group chosen from among methyl, ethyl, propyl groups; an aryl group chosen from among benzyl and phenyl groups.

**6.** Method according to any one of claims 1 to 5, **characterised in that** the depolymerisation of lignin implements a lignin containing at least 30% of cleavable bonds with respect to the total number of bonds present between the monomer units in the lignin.

**7.** Method according to any one of claims 1 to 6, **characterised in that** the depolymerisation of lignin implements a lignin containing at least 30% of bond of the type β-O-4 and/or at least 3% of α-O-4 bonds, the percentages indicated corresponding to the percentage of a type of bond with respect to the total number of bonds present between the monomer units in the lignin.

**8.** Method according to any one of claims 1 to 7, **characterised in that** the depolymerisation of lignin implements an organic catalyst chosen from among:

- the carbocations chosen from among trityl cation ($(C_6H_5)_3C^+$), tropilium $(C_7H_7)^+$, benzylic cation ($C_6H_5CH_2^+$), allylic cation ($CH_3$-$CH^+$-$CH$=$CH_2$), methylium ($CH_3^+$), cyclopropylium ($C_3H_5^+$), cyclopropyl carbocation chosen from among cyclopropyl dimethyl carbocation and dicyclopropyl carbocation, triazabicyclodecene cation (TBD), acylium ($R^1$-C=O)$^+$ with $R^1$ chosen from among methyl, propyl and benzyl, benzenium $(C_6H_5)^+$, norbornyl cation $(C_7H_{11})^+$;
- oxoniums chosen from among $(CH_3)_3O^+BF_4^-$ and $(CH3CH_2)_3O^+BF_4^-$;
- a silylium ion chosen from among $Et_3Si^+$ and $Me_3Si^+$;
- disilyl cations having a bridging hydride chosen from among the formulas indicated below

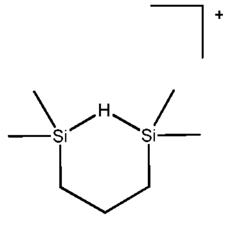 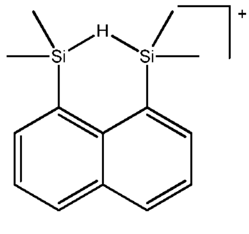 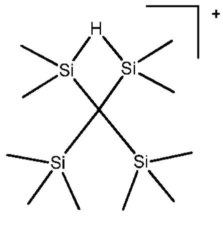

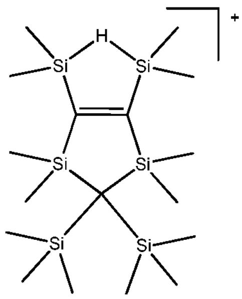

with the counterion of said silylium ion, said carbocations and said disilyl cations being

- a halide chosen from among F-, $Cl^-$, $Br^-$ and $I^-$; or
- an anion chosen from among $BF4^-$, $SbF_6^-$, $B(C_6F_5)_4^-$, $B(C_6H_5)_4^-$, $CF_3SO_3^-$, $PF_6^-$.

**9.** Method according to any one of claims 1 to 7, **characterised in that** the depolymerisation of lignin implements an organometallic catalyst chosen from among:

- iridium complexes of formula (IV),

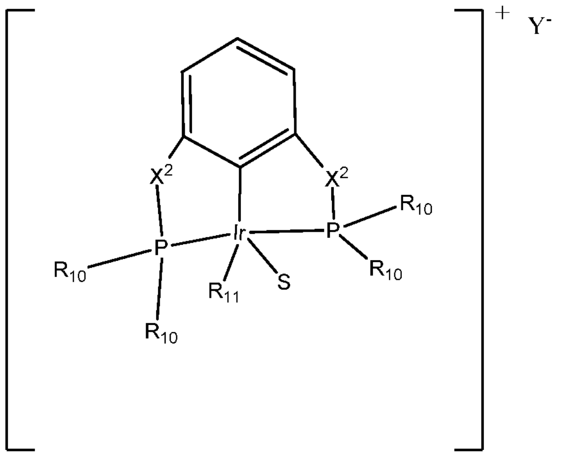

(IV)

wherein

- $R^{10}$ represents an alkyl or aryl group;
- $R^{11}$ represents a hydrogen atom or an alkyl group;
- $X^2$ represents a group $-CH_2-$ or an oxygen atom;
- $Y^-$ represents a counterion chosen from among $B(C_6F_5)_4^-$ and $B(C_6H_5)_4^-$; and
- S represents a solvent molecule, coordinated to the complex, chosen from among dimethyl sulfoxide (DMSO), acetonitrile ($CH_3CN$) and acetone ($CH_3COCH_3$); and
- ruthenium complexes of formula (V)

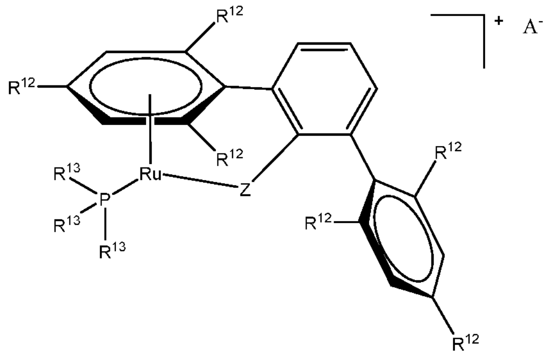

(V)

wherein

- $R^{12}$ represents a hydrogen atom or an alkyl group;
- $R^{13}$ represents an aryl or an alkyl group, said aryl and alkyl groups being optionally substituted;
- Z represents a group $-CH_2-$, an oxygen atom or a sulphur atom; and
- $A^-$ represents a counterion chosen from among $B(C_6F_5)_4^-$ and $[CHB_{11}H_5Cl_6]^-$.

**10.** Method according to claim 9, **characterised in that** the organometallic catalyst is chosen from among:

- the iridium complex $[(POCOP)Ir(H)(acetone)]^+B(C_6F_5)_4^-$ with (POCOP) representing 2,6-bis(di-tert-butylphosphinito)phenyl; and
- ruthenium complexes of formula (V) wherein
    - $R^{12}$ represents a methyl group;
    - $R^{13}$ represents *p*-$FC_6H_4$;
    - Z represents a sulphur atom; and
    - $A^-$ represents $B(C_6F_5)_4^-$.

**11.** Method according to any one of claims 1 to 7, **characterised in that** the depolymerisation of lignin implements an organometallic and metallic catalyst chosen from among:

- boron compounds chosen from among $BF_3$, $BF_3(Et_2O)$, $BCl_3$, $BBr_3$, hydroborane triphenyl, hydroborane tricyclohexyl, $B(C_6F_5)_3$, B-methoxy-9-borabicyclo[3.3.1]nonane (B-methoxy-9-BBN), B-benzyl-9-borabicyclo[3.3.1]nonane (B-benzyl-9-BBN);
- the borenium compound Me-TBD-$BBN^+$, ferrocene borenium derivatives responding to the formula

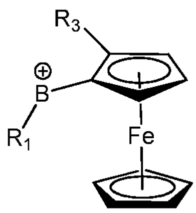

borenium ferrocène

- wherein $R^1$= phenyl and $R^3$= 3,5-dimethylpyridyl;
- the compounds of aluminium chosen from among $AlCl_3$, $AlBr_3$, aluminium isopropoxide $Al(O\text{-}i\text{-}Pr)_3$, aluminium ethanoate $(Al(C_2H_3O_2))$, Krossing salt $[Ag(CH_2Cl_2)]\{Al[OC(CF_3)_3]_4\}$, Li{Al[OC(CF3)3]4}, $Et_2Al^+$;
- indium compounds chosen from among $InCl_3$, $In(OTf)_3$;
- iron compounds chosen from among $FeCl_3$, $Fe(OTf)_3$;
- tin compounds chosen from among $SnCl_4$, $Sn(OTf)_2$;
- phosphorus compounds chosen from among $PCl_3$, $PCl_5$, $POCl_3$;
- transition metal trifluoromethanesulfonate or triflate compounds $(CF_3SO_3^-)$ and lanthanides chosen from among

scandium triflate, ytterbium triflate, yttrium triflate, cerium triflate, samarium triflate, neodymium triflate.

**12.** Method according to claim 11, **characterised in that** the catalyst is chosen from among $BF_3$; $InCl_3$; triphenylcarbenium tetrakis(perfluorophenyl)borate $[(Ph)_3C^+B(C_6F_5)_4^-$,$B(C_6F_5)_3]$.

**13.** Method according to any one of claims 1 to 12, **characterised in that** in the depolymerisation step, the reaction is carried out:

- under a pressure of one or of a mixture of inert gas(es) chosen from among nitrogen and argon, or gases generated by the method, in particular, of methane and hydrogen comprised between 0.2 and 50 bars, limits included;
- at a temperature comprised between 0 and 150°C, limits included;
- in one or a mixture of at least two solvent(s) chosen from among:

  - silyl ethers chosen from among 1,1,1,3,3,3-hexamethyldisiloxane ($(Me_3Si)_2O$), 1,1,1,3,3,3-hexaethyldisiloxane ($(Et_3Si)_2O$);
  - hydrocarbons chosen from among benzene, toluene, pentane and hexane;
  - sulfoxides chosen from among dimethyl sulfoxide (DMSO);
  - alkyl halides chosen from among chloroform, methylene chloride, chlorobenzene, dichlorobenzene.

**14.** Method according to any one of claims 1 to 13, **characterised in that** in the depolymerisation step, the mass ratio between the silane compound of formula (III) and the lignin is comprised between 0.5 and 6, limits included.

**15.** Method according to any one of claims 1 to 14, **characterised in that** the quantity of catalyst is of 0.001 to 1 mass equivalent, limits included, with respect to the initial mass of lignin.

**16.** Method according to any one of claims 1 to 15, **characterised in that** prior to the step of depolymerising lignin, the plant species from which the lignin comes is selected so as to have:

- at least 10% by mass of lignin with respect to the total mass of the sample of the plant species selected;
- at least 30% of cleavable bonds with respect to the total number of bonds present between the monomer units in the lignin; and
- at least 50% of G, H or S residue from the total number of residues present in the lignin used.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- US 4151207 A
- WO 2011003029 A


### Littérature non-brevet citée dans la description


- **F. G. CALVO-FLORES ; J. A. DOBADO.** *ChemSusChem.,* 2010, vol. 3, 1227-1235
- Methods in Lignin Chemistry. **S. Y. LIN.** Springer Series in Wood Science. Springer, 1992
- **R. VANHOLME ; K. MORREEL ; J. R. W. BOERJAN.** *Curr. Opin. Plant Biol.,* 2008, vol. 11, 278-285
- **F. G. CALVO-FLORES ; J. A. DOBADO.** *ChemSusChem,* 2010, vol. 3, 1227-1235
- **E. ADLER.** *Wood Sci. Technol.,* 1977, vol. 11, 169
- **M. P. PANDEY ; C. S. KIM.** *Chem. Eng. Technol,* 2011, vol. 34, 29
- **M. B. HOCKING.** *J. Chem. Educ.,* 1997, vol. 74, 1055-1059
- **F. G. CALVO-FLORES ; J. A. DOBADO.** *ChemSusChem.,* 2010, vol. 3, 1227-1235
- **J. ZHANG ; Y. CHEN ; M.A.BROOK.** *Acs sustainable Chemistry and engineering,* 2014, vol. 2, 1983-1991
- **J. ZAKZESKI ; P. C. A. BRUIJNINCX ; A. L. JONGERIUS ; B. M. WECKHUYSEN.** *Chem Rev.,* 2010, vol. 110, 3552
- **J. M. NICHOLS ; L. M. BISHOP ; R. G. BERGMAN ; J. A. ELLMAN.** *J. Am. Chem. Soc,* 2010, vol. 132, 12554-12555
- **A. WU ; B. O. PATRICK ; E. CHUNG ; B. R. JAMES.** *Dalton Trans.,* 2012, vol. 41, 11093
- **T. VOM STEIN ; T. WEIGAND ; C. MERKENS ; JURGEN KLANKERMAYER ; W. LEITNER.** *ChemCatChem,* 2013, vol. 5, 439-441
- **S. SON ; F. D. TOSTE.** *Angew. Chem. Int. Ed.,* 2010, vol. 49, 3791-3794
- **A. G. SERGEEV ; J. F. HARTWIG.** *Science,* 2011, vol. 332, 439
- **E. FEGHALI ; T. CANTAT.** *Chem. Commun,* 2014, vol. 50, 862-865
- **J. M. W. CHAN ; S. BAUER ; H. SOREK ; S. SREEKUMAR ; K. WANG ; F. D. TOSTE.** *ACS Catal,* 2013, vol. 3, 1369-1377
- **M. NAGY ; K. DAVID ; G. J. P. BRITOVSEK ; A. J. RAGAUSKAS.** *Holzforschung,* 2009, vol. 63, 513
- **L.B. DAVIN, L.B. ; N. G. LEWIS.** *Curr, Opin. Biotechnol.,* 2005, vol. 16, 407-415
- **P. J. DEUSS ; K. BARTA ; J. G. DE VRIES.** *Cata/. Sci. Technol.,* 2014
- **R. R. NAREDLA ; D. A. KLUMPP.** *Chem. Rev.,* 2013, vol. 113, 6905-6948
- **M. SAUNDERS. ; H. A. JIMENEZ-VAZQUEZ.** *Chem. Rev.,* 1991, vol. 91, 375-397
- **I. GOTTKER-SCHNETMANN ; P. WHITE ; M. BROOKHART.** *J. Am. Chem. Soc.,* 2004, vol. 126, 1804-1811
- **J. YANG ; M. BROOKHART.** *J. Am. Chem. Soc.,* 2007, vol. 129, 12656-12657
- **T. STAHL ; H. F. T. KLARE ; M. OESTREICH.** *J. Am. Chem. Soc.,* 2013, vol. 135, 1248-1251
- **Y. SHOJI ; N. TANAKA ; K. MIKAMI ; M. UCHIYAMA ; T. FUKUSHIMA.** *Nat. Chem.,* 2014, vol. 6, 498-503
- **J. CHEN ; R. A. LALANCETTEA ; F. JÄKLE.** *Chem. Commun.,* 2013, vol. 49, 4893-4895
- **I. KROSSING.** *Chem.-Eur. J.,* 2001, vol. 7, 490
- **M. KHANDELWAL ; R. J. WEHMSCHULTE.** *Angew. Chem. Int. Ed.,* 2012, vol. 51, 7323-7326
- **J. J. STEWART ; T. AKIYAMA ; C. CHAPPLE ; J. RALPH ; S. D. MANSFIELD.** *Plant Physiol.,* 2009, vol. 150, 621-635
- **J, H. LORA ; W. G. GLASSER.** *J Polym Environ,* 2002, vol. 10, 39-48
- **BOJAN JANKOVIC.** *Bioresource Technol.,* 2011, vol. 102, 9763-9771
- **J. C. PARAJO ; J. L. ALONSO ; D. VAZQUEZ.** *Bioresource Technology,* 1993, vol. 46, 233-240
- **I. MIRANDA ; H. PEREIRA.** *Holzforschung,* 2002, vol. 56, 85-90
- **A. JOHANSSON ; O. AALTONEN ; P. YLINEN.** *Biomass,* 1987, vol. 13, 45-65
- **X.F. SUN ; R.C. SUN ; P. FOWLER ; M.S. BAIRD.** *Carbohydr. Polym.,* 2004, vol. 55, 379-391
- **P. LIGERO ; A. VEGA ; J.J. VILLAVERDE.** *Bioresource Technol.,* 2010, vol. 101, 3188-3193

- **A. LINDNER ; G. WEGENER.** *J. Wood Chem. Technol.,* 1988, vol. 8, 323-340
- **J. H. LORA ; W. G. GLASSER.** *J Polym Environ,* 2002, vol. 10, 39-48
- **P. LIGERO ; A. VEGA ; J.J. VILLAVERDE.** *Bioresource Technol,* 2010, vol. 101, 3188-3193
- **H. Q. LAM ; Y. LE BIGOT ; M. DELMAS ; G. AVIGNON.** *Industrial Crops and Products,* 2001, vol. 14, 139-144